(19) Europäisches Patentamt
European Patent Office
Office européen des brevets

(11) Veröffentlichungsnummer: **0 003 212**
**B1**

(12) **EUROPÄISCHE PATENTSCHRIFT**

(45) Veröffentlichungstag der Patentschrift: **17.03.82**

(21) Anmeldenummer: **78101300.8**

(22) Anmeldetag: **03.11.78**

(51) Int. Cl.³: **C 07 D 239/48,**
A 61 K 31/505,
C 07 D 403/10

(54) **2.4-Diamino-5-benzylpyrimidine und diese enthaltende pharmazeutische Präparate sowie deren Herstellung.**

(30) Priorität: **10.11.77 LU 78487**
**19.09.78 CH 9776/78**

(43) Veröffentlichungstag der Anmeldung:
**08.08.79 Patentblatt 79/16**

(45) Bekanntmachung des Hinweises auf die Patenterteilung
**17.03.82 Patentblatt 82/11**

(84) Benannte Vertragsstaaten:
**BE CH DE FR LU NL SE**

(56) Entgegenhaltungen:
**DE - A - 2 202 715**
**DE - A - 2 452 889**
**DE - A - 2 558 150**
**DE - A - 2 635 765**

**JOURNAL OF MEDICINAL CHEMISTRY, vol. 17, Nr. 3, 1974**
**B. P. DAS et al.: "Synthesis of 2,4-Diamino-5-[4-arylthiophenyl]-an 5-[4-Arylsulfonylphenyl] pyrimidines as Antimalarials"**
**Seite 372 bis 374**

(73) Patentinhaber: **F. HOFFMANN-LA ROCHE & CO. Aktiengesellschaft**
**CH-4002 Basel (CH)**

(72) Erfinder: **Kompis, Ivan, Dr.**
**Lettenhofstrasse 6**
**CH-4104 Oberwil (CH)**
Erfinder: **Wick, Alexander Eduard, Dr.**
**Mühlestiegstrasse 34**
**CH-4125 Riehen (CH)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 212 B1

2.4-Diamino-5-benzylpyrimidine und diese enthaltende pharmazeutische Präparate sowie deren Herstellung

Die vorliegende Erfindung betrifft neue 2,4-Diamino-5-benzylpyrimidine de allgemeinen Formel

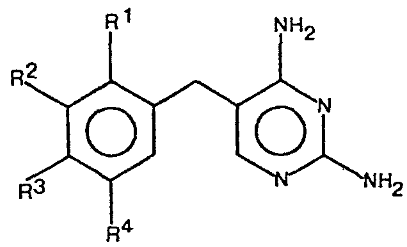

I

worin $R^1$ Wasserstoff oder $C_{1-6}$-Alkyl; $R^2$ Wasserstoff und $R^3$ und $R^4$ $C_{1-6}$-Alkylthio oder $R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder $R^1$ Wasserstoff, $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen und deren physiologisch verträgliche Salze, Verfahren zu deren Herstellung, sowie pharmazeutische Präparate auf der Basis dieser Verbindungen und deren Herstellung.

Die allgemeine Formel I umfasst die Verbindungsgruppen der Formeln I—1, I—2 und I—3:

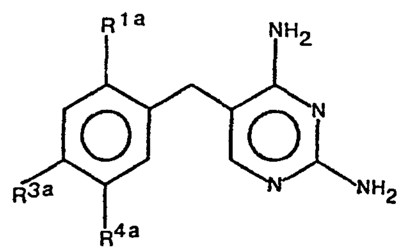

I-1

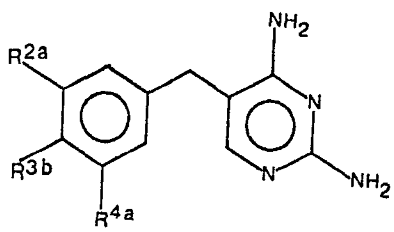

I-2

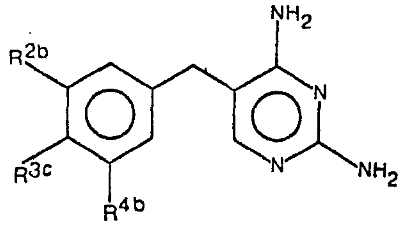

I-3

In den Formeln I—1, I—2 und I—3 bedeuten:

- $R^{1a}$ Wasserstoff oder $C_{1-6}$-Alkyl;
- $R^{2a}$ $C_{1-6}$-Alkylthio;
- $R^{2b}$ $C_{1-6}$-Alkoxy;
- $R^{3a}$ $C_{1-6}$-Alkylthio;
- $R^{3b}C_{1-6}$-Alkoxy, $C_{1-6}$-Alkylthio oder Pyrrolo;
- $R^{3c}$ $C_{1-6}$-Alkylthio, Carboxy-$C_{1-6}$-alkylthio oder Cyano-$_{1-6}$-alkylthio;
- $R^{4a}$ $C_{1-6}$-Alkylthio;
- $R^{4b}$ $C_{1-6}$-Alkoxy.

Beispiele für $C_{1-6}$-Alkylreste sind Methyl, Aethyl, Propyl, Isopropyl, Butyl, sek.-Butyl, Isobutyl, tert.-Butyl, Pentyl und Hexyl, wobei Methyl und Aethyl bevorzugt sind.

Das Verfahren zur Herstellung der erfindungsgemässen Verbindungen ist dadurch gekennzeichnet, dass man
(a) eine Verbindung der Formel

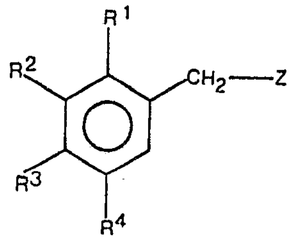

II

worin $R^1$, $R^2$, $R^3$ und $R^4$ die oben angegebenen Bedeutungen haben, Z eine Gruppe $-C(CN)=CH-Y$ oder $-CH(CN)CH(OR^5)_2$, Y eine Abgangsgruppe und $R^5$ $C_{1-6}$-Alkyl, 2—$C_{1-6}$-Alkoxy-äthyl, 2-Phenoxyäthyl oder 2-Benzyloxy-äthyl oder beide Reste $R^5$ gemeinsam $C_{1-6}$-Alkylen darstellen, mit Guanidin oder einem Guanidinsalz kondensiert oder
(b) aus einer Verbindung der Formel

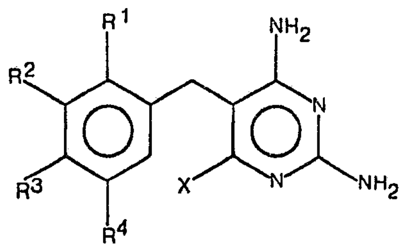

III

worin X Halogen darstellt und $R^1$, $R^2$, $R^3$ und $R^4$ die oben angegebenen Bedeutungen haben, den Substituenten X reduktiv eliminiert oder
(c) zur Herstellung einer Verbindung der Formel I—1 ein substituiertes Benzylpyrimidin der Formel

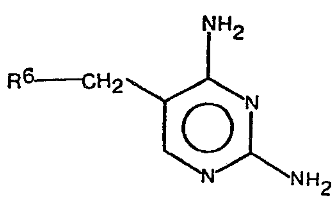

IV

worin $R^6$ $C_{1-6}$-Alkoxy (vorzugsweise Methoxy), Benzyloxy, Hydroxy oder Halogen darstellt, mit einer Verbindung der Formel

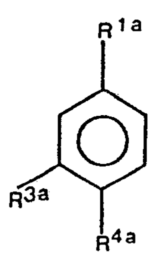

V

worin $R^{1a}$, $R^{3a}$ und $R^{4a}$ die vorstehend angegebenen Bedeutungen umsetzt haben, und die erhaltene Verbindung gewünschtenfalls in ein physiologisch verträgliches Salz überführt.

Gemäss Variante (a) des erfindungsgemässen Verfahrens wird eine Verbindung II mit Guanidin oder einem Guanidinsalz umgesetzt. Die Umsetzung kann in an sich bekannter Weise erfolgen, z.B. in einem organischen Lösungsmittel, wie einem Alkanol (Methanol, Aethanol), in Dimethylformamid, Dimethylsulfoxid oder N-Methylpyrazolon, bei einer Temperatur ziwschen 25 und 200°, vorzugsweise zwischen 50 und 170°. Als Guanidinsalze können beispielsweise das Carbonat und das Hydrochlorid verwendet werden. Repräsentative Beispiele für durch das Symbol Y in der Formel II dargestellte Abgangsgruppen sind z.B. Alkoxygruppen, wie Methoxy, Aethoxy, Propoxy etc., 2—($C_{1-6}$-Alkoxy)-äthoxygruppen, wie 2-Methoxy-äthoxy und 2-Aethoxy-äthoxy, 2-Phenoxy-äthoxy, 2-Benzyloxy-äthoxy, Alkylthiogruppen, die Aminogruppe und durch aliphatische, aromatische oder heterocyclische Gruppen substituierte Aminogruppen wie Alkylamino, Benzylamino, Arylamino (z.B. gegebenenfalls substituiertes Anilino oder Naphthylamino), Dialkylamino, 1-Imidazolyl, Pyrrolidino, Piperidino, Piperazino oder Morpholino. Besonders bevorzugt als Abgangsgruppe ist der Anilinorest, dessen Phenylring gegebenenfalls ein- oder mehrfach durch Halogen, Alkyl oder Alkoxy substituiert sein kann.

Die Herstellung der Ausgangsverbindungen II kann, wie im Reaktionsschema I dargestellt, aus Verbindungen der Formel VIII erfolgen, wobei die einzelnen Reaktionsschritte in Analogie zu bekannten Umsetzungen erfolgen können.

So kann z.B. ein Aldehyd der Formel VIII mit einem $\beta$-Alkoxypropionitril der Formel $NC—CH_2—CH_2—OR^5$ zu einer Verbindung der Formel VI und in Gegenwart eines Alkalimetalloxides wie Natriummethoxid oder Kalium-tert.-butylat und eines Alkanols wie Methanol, Aethanol oder Propanol zu einer Verbindung der Formel II-a umgesetzt werden. Die $\alpha,\beta$-ungesättigte Verbindung VI steht im Gleichgewicht mit der entsprechenden $\beta,\gamma$-ungesättigten Verbindung II-e, die mit Guanidin zu einer Verbindung I umgesetzt werden kann.

Setzt man einen Aldehyd der Formel VIII zunächst mit Malodinitril unter den Bedingungen einer Knoevenagel-Kondensation (ggf. basischer Katalysator) um, so erhält man ein Dinitril der Formel VII, das durch selektive Hydrierung, z.B. mit einem halben Mol Natriumborhydrid, in die Verbindung II-b übergeführt werden kann.

Durch Kondensation mit $\beta$-Morpholinopropionitril kann ein Aldehyd der Formel VIII in eine Verbindung der Formel II-c übergeführt werden, die entweder direkt als Ausgangsmaterial für die erfindungsgemässe Umsetzung dienen kann, oder aber, und zwar vorzugsweise, zunächst noch durch Austausch der Morpholino- gegen die Anilinogruppe in eine Verbindung der Formel II-d übergeführt wird.

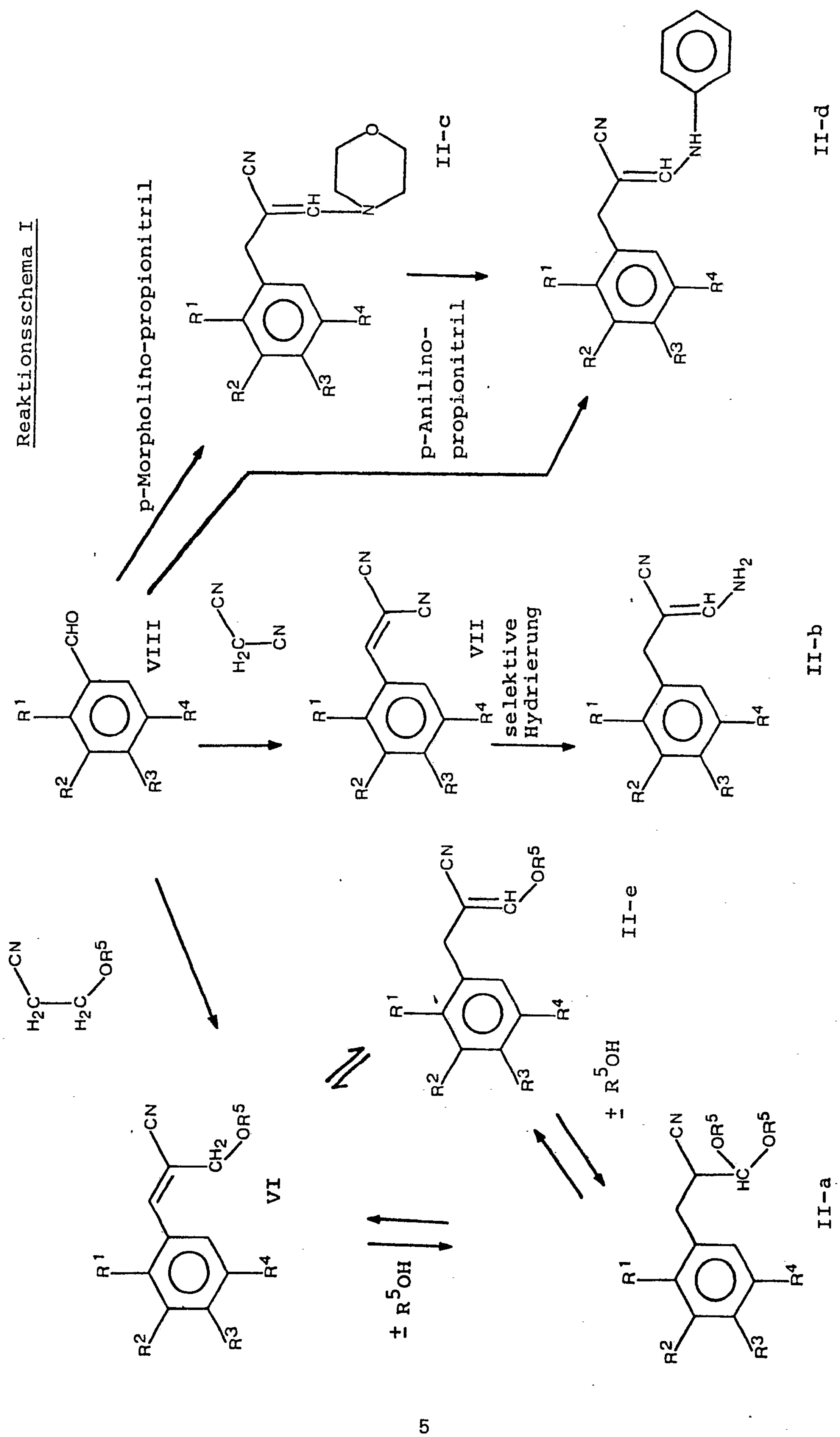
Reaktionsschema I
VIII
p-Morpholino-propionitril
II-c
p-Anilino-
propionitril
II-d
VII
selektive
Hydrierung
II-b
II-e
VI
II-a

Die Umsetzung von VIII zu II-d kann auch direkt mit $\beta$-Anilinoproprionitril erfolgen, doch stellt die Morpholino-Anilino-Variante den zweckmässigsten Weg zur Herstellung der Benzylpyrimidine I dar, weil hierbei unter milderen Bedingungen bessere Ausbeuten erzielt werden.

Die Methoden der Herstellung der erfindungsgemässen schwefelhaltigen Benzylpyrimidine aus Aldehyden der Formel I sind somit im Prinzip denen analog, die für die Herstellung entsprechender nicht schwefelhaltiger Benzylpyrimidine bekannt sind.

Die Aldehyde der Formel VIII sind, mit Ausnahme des 3,4-Bis-(methylthio)-benzaldehyde (d.i. Verb. VIII—1 mit $R^{1a}$ = Wasserstoff und $R^{3a} = R^{4a}$ = Methylthio; DE—A—22 02 715) neue Verbindungen. In Analogie zu der Unterteilung der Verbindungen I können die Aldehyde VIII in die folgenden Untergruppen VIII—1, VIII—2 und VIII—3 aufgeteilt werden:

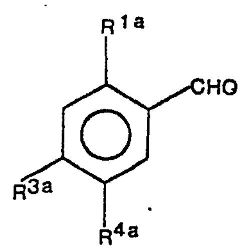

VIII-1

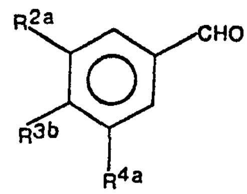

VIII-2

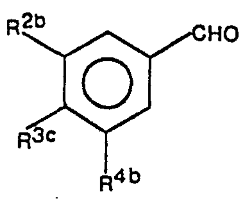

VIII-3

In den Formeln VIII—1, VIII—2 und VIII—3 haben die sie Substituenten die bereits vorstehend angegebenen Bedeutungen.

Die Herstellung der Aldehyde der Formel VIII aus bekannten Verbindungen kann nach an sich bekannten Methoden, z.B. wie in Beispiel 7 im Detail beschrieben und wie in den folgenden Reaktionsübersichten II—IV schematisch dargestellt, erfolgen.

Eine Reaktion, für die es in der Literatur bisher keine Parallele gibt und die daher neu ist, stellt die Thermolyse einer Verbindung XXIII zu einer Verbindung VIII—3c dar (vgl. Reaktionsschema IV). Die Reaktion wird zweckmässigerweise in Dimethylformamid bei einer Temperatur von ca. 50°C bis zur Rückflusstemperatur des Reaktionsgemisches durchgeführt. Durch Spuren von Alkalimetallxanthogenat, beispielsweise Kaliumxanthogenat, wird die Reaktion katalysiert.

Die reduktive Eliminierung des Halogenatoms X, das vorzugsweise ein Chlor- oder Bromatom ist, in einer Verbindung der Formel III gemäss Verfahrensvariante (B), kann in an sich bekannter Weise durch Behandlung einer Verbindung III mit geeigneten Reduktionsmitteln wie Zink in Eisessig, amalgamiertem Zink in Natronlauge oder durch katalytische Hydrierung, beispielsweise in Gegenwart eines Edelmetallkatalysators, wie Palladium, in einem inerten Lösungsmittel wie Aethanol, erfolgen.

Die Verbindungen der Formel III sind neu. Ihre Herstellung kann in Analogie zu bekannten Verfahren erfolgen, die im folgenden Reaktionsschema V zusammengefasst sind.

Reaktionsschema II

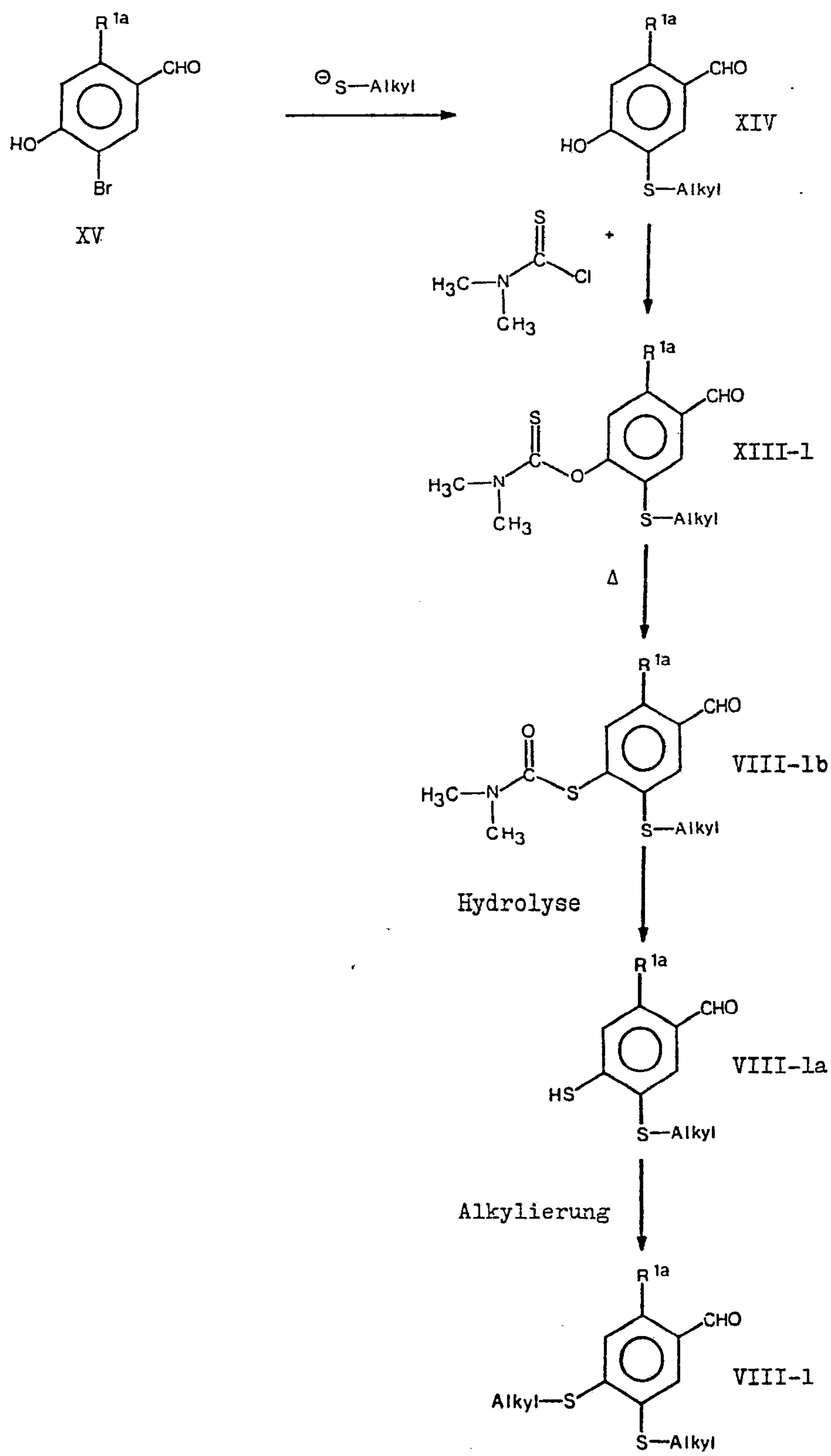


XV

XIV

XIII-1

VIII-1b

VIII-1a

VIII-1

Reaktionsschema III

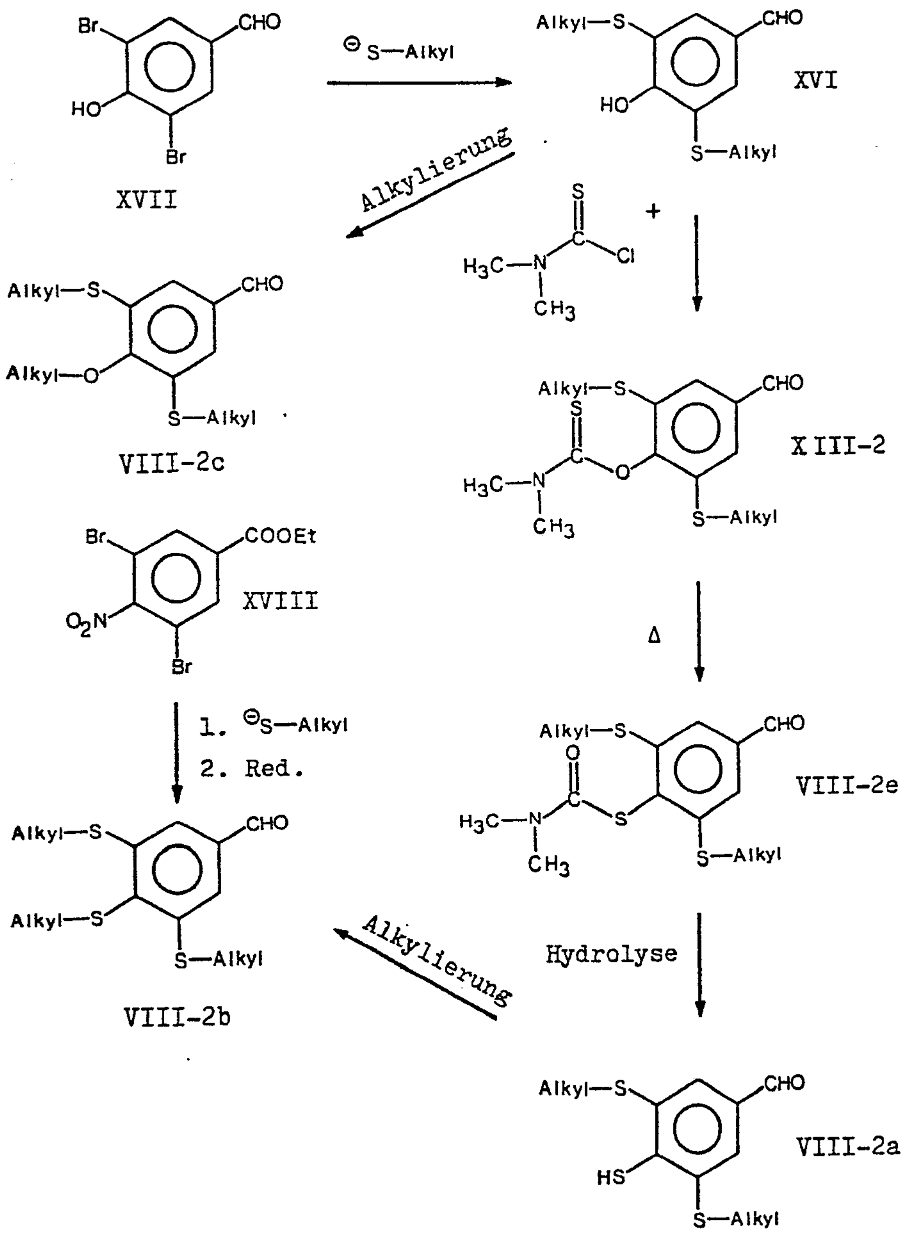


XVII, XVI, VIII-2c, XIII-2, XVIII, VIII-2e, VIII-2b, VIII-2a

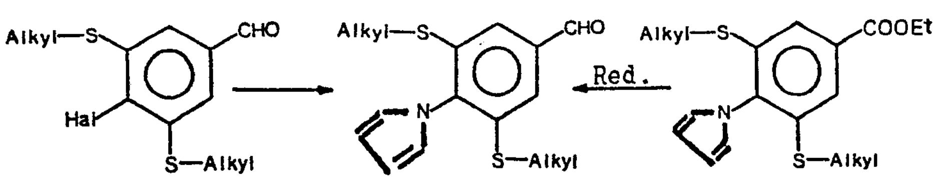


XX VIII-2f XIX

Reaktionsschema IV

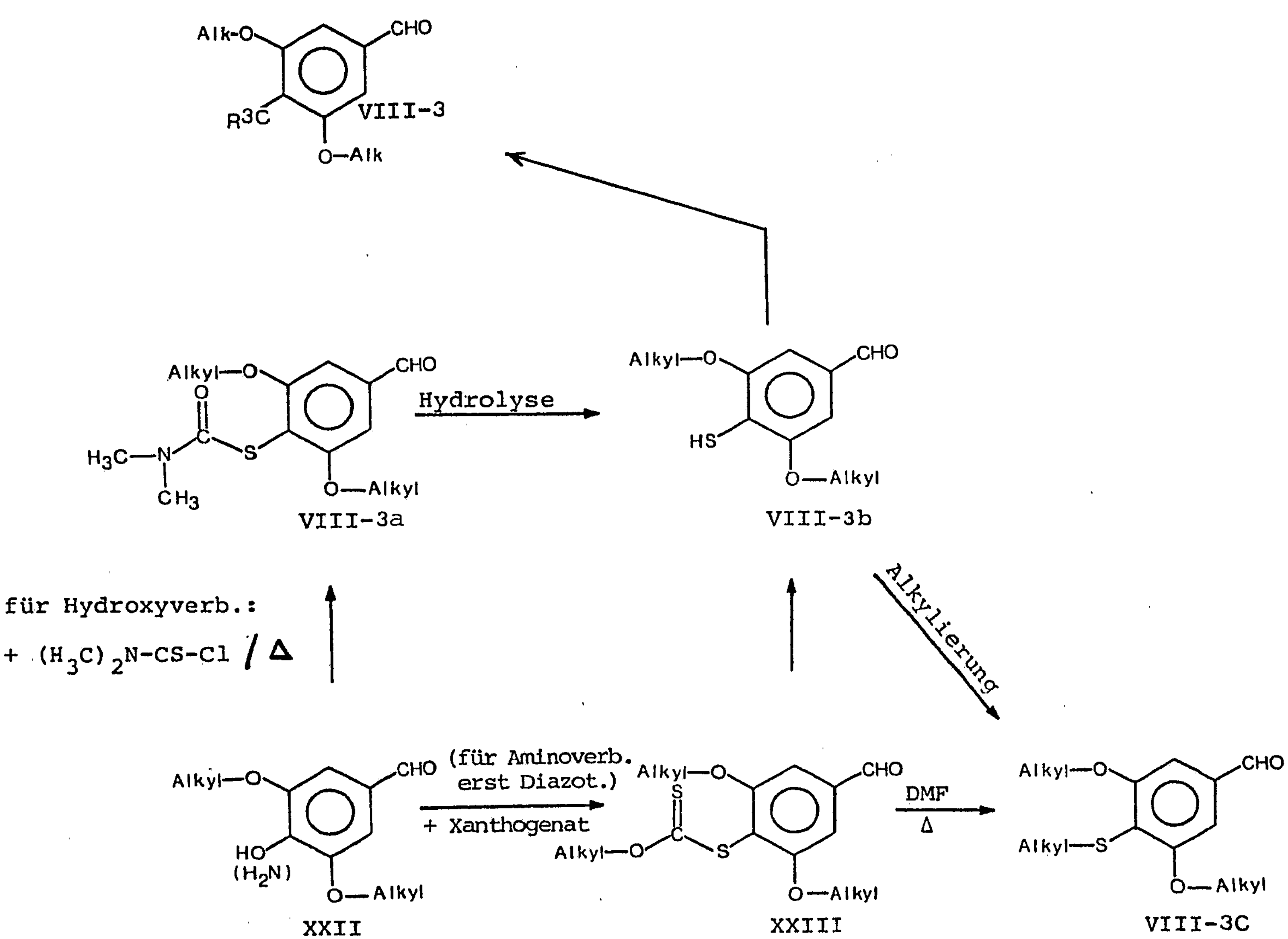


VIII-3

VIII-3a

VIII-3b

XXII

XXIII

VIII-3C

Reaktionsschema V

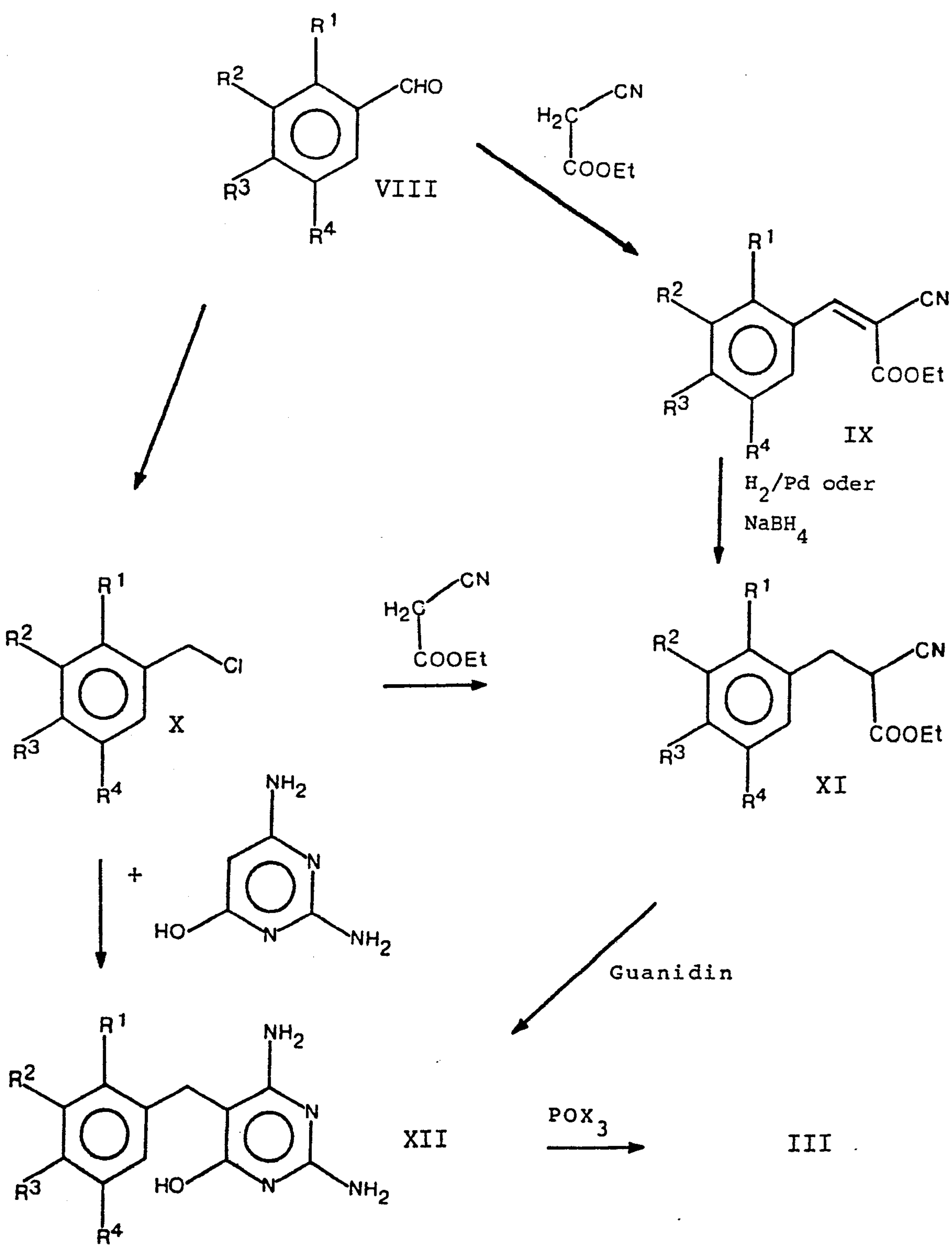

Die Umsetzung einer Verbindung IV mit einer Verbindung V gemäss Verfahrensvariante (c) kann in Gegenwart einer anorganischen Säure, beispielsweise Orthophosphorsäure, Polyphosphorsäure, einer Halogenwasserstoffsäure, wie Chlorwasserstoffsäure oder Bromwasserstoffsäure, oder einer organischen Säure, beispielsweise einer Trihalogenessigsäure, wie Trifluoressigsäure, erfolgen.

Die Reaktion wird zweckmässigerweise in einem Temperaturbereich von etwa 50 bis etwa 100°C ausgeführt. Im allgemeinen kann die für die Reaktion verwendete anorganische oder organische Säure als Lösungsmittel dienen, jedoch kann gewünschtenfalls auch ein inertes organisches Lösungsmittel verwendet werden. Das erhaltene Säureadditionssalz einer Verbindung der Formel I—1 wird im Anschluss an die Reaktion in üblicher Weise abgetrennt und durch konventionelle Reinigungsmethoden, wie Kristallisation oder Filtration, gereinigt. Die freie Base kann durch Neutralisation des Säureadditionssalze, beispielsweise mit einem Alkalimetallhydroxid erhalten werden.

Während die Ausgangsverbindungen der Formel IV bekannt sind, handelt es sich bei den Verbindungen der Formel V um teilweise neue Verbindungen. Die neuen Verbindungen lassen sich in an sich bekannter Weise aus bekannten Verbindungen, z.B. dem 1,2-Dimercaptobenzol, dem 2-Methylmercaptobenzol, dem 3,4-Dimercaptotoluol oder dem 3,4-Bis-(methylthio)-toluol herstellen oder in Analogie zu der Herstellung dieser bekannten Verbindungen, beispielsweise durch Alkylierung einer Mercaptogruppe und/oder Oxidation einer Alkylthiogruppe zu einer Alkylsulfinyl oder Alkylsulfonylgruppe. Die Herstellung einer Carboxyalkylthioverbindung kann z.B. durch Umsetzung einer Mercaptoverbindung mit einem $\omega$-Halogencarbonsäurederivat, wie einem Ester oder Amid, und nachfolgender Hydrolyse erfolgen.

Als Salze der Verbindungen I kommen sowohl Salze mit Säuren (sog. Säureadditionssalze) als auch, je nach Substituent am Benzylrest, Salze mit Basen in Frage.

Für die Herstellung von Säureadditionssalzen, insbesondere von in pharmazeutischen Präparaten verwendbaren, d.h. physiologisch verträglichen Salzen, kommen die üblicherweise für diesen Zweck verwendeten anorganischen und organischen Säuren in Betracht, wie Salzsäure, Schwefelsäure, Phosphorsäure, Ameisensäure, Essigsäure, Oxalsäure, Weinsäure, Maleinsäure, Benzoesäure, Bernsteinsäure, Fumarsäure, Lävulinsäure, Salicylsäure, Citronensäure, Isocitronensäure, Adipinsäure, Milchsäure, $\alpha$-Ketoglutarsäure, Aepfelsäure, Malonsäure, Glycerinsäure, Mevalonsäure, Glucuronsäure, Neuraminsäure, Glutarsäure, Glucarsäure, Asparaginsäure, Gluconsäure, Mandelsäure, Ascorbinsäure, Lactobionsäure, Glucoheptonsäure, Glutaminsäure, Nicotinsäure, Panthotensäure, Folsäure, Adenylsäure, Geranylsäure, Cytidinsäure, Inosinsäure und Methansulfonsäure.

Beispiele von Salzen mit Basen sind das Natrium- und Kaliumsalz.

Die Verbindungen der Formel I und ihre Salze sind antibakteriell wirksam. Sie hemmen die bakterielle Dihydrofolsäurereduktase (DHFR) und potenzieren darüberhinaus die Wirkung von Sulfonamiden und anderen Dihydrofolsäuresynthetasehemmern. Beispiele von Sulfonamiden, die durch die erfindungsgemässen Verbindungen potenziert werden, sind solche der Pyrimidin-, Isoxazol-, Oxazol- und Pyrazinreihe wie Sulfadiazin, Sulfadimethoxin, Sulfadoxin, Sulfamerazin, Sulfameter, Sulfamethazin, 6-Methoxy-4-sulfanilamidopyrimidin, Sulfamethoxazol, Sulfisoxazol, 3-Sulfanilamido-4,5-dimethylisoxazol, Sulfamoxol und Sulfalen.

Obgleich ähnliche 2,4-Diamino-5-benzylpyrimidine seit langem bekannt sind (Trimethoprim, Ormetoprim, Diaveridin) und es seither an Versuchen nicht gefehlt hat, durch Variation der Substituenten, vor allem am Phenylrest, weitere gut oder besser wirksame Verbindungen gleicher Wirkungsrichtung zu finden und zu entwickeln (siehe z.B. die deutschen Offenlegungsschriften Nrn. 22 02 715, 24 52 889, 25 58 150 und 26 35 765), sind die vorliegenden Verbindungen die ersten, bei denen ein Ersatz von Sauerstoff durch Schwefel vorgenommen wurde. Es ist daher davon auszugehen, das ein solcher Ersatz für den Fachman nicht naheliegend war. Dass die erfindungsgemässen Verbindungen den strukturell nächststehenden bekannten gut wirksamen Verbindungen ebenbürtig, weitgehend sogar überlegen sind (niedrigere 50%ige Hemmkonzentrationen der bakteriellen DHFR, beispielsweise bei E. coli; geringere Toxizitäten), wird durch die folgende Tabelle belegt.

TABELLE 1

| Verbindung (Beisp. No.) | $R^1$ | $R^2$ | $R^3$ | $R^4$ | 50% Hemmung der DHFR ($M \times 10^6$) E. coli |
|---|---|---|---|---|---|
| Trimethoprim | H | $OCH_3$ | $OCH_3$ | $OCH_3$ | 0,9 |
| 1 | H | $OCH_3$ | $SCH_3$ | $OCH_3$ | 0,35 |
| 2 | H | $OCH_3$ | $SC_2H_5$ | $OCH_3$ | 0,18 |
| 2 | H | $SCH_3$ | $OCH_3$ | $SCH_3$ | 0,72 |
| 2 | H | $SCH_3$ | $SCH_3$ | $SCH_3$ | 0,45 |
| 2 | H | $SCH_3$ | 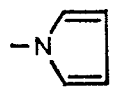 | $SCH_3$ | 0,52 |
| Diaveridin | H | H | $OCH_3$ | $OCH_3$ | 10 |
| 2 | H | H | $SCH_3$ | $SCH_3$ | 1,6 |
| Ormetoprim | $CH_3$ | H | $OCH_3$ | $OCH_3$ | 6,7 |
| 2 | $CH_3$ | H | $SCH_3$ | $SCH_3$ | 1,5 |

Die erfindungsgemässen Benzylpyrimidine können daher als pharmazeutische Präparate mit direkter oder verzögerter Freigabe des Wirkstoffes in Mischung mit einem für die orale, rektale oder parenterale Applikation geeigneten organischen oder anorganischen inerten Trägermaterial, z.B. Wasser, Gelatine, Gummi arabicum, Milchzucker, Stärke, Magnesiumstearat, Talk, pflanzlichen Oelen, Polyalkylenglykolen, Vaseline, usw. verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der jedem Fachmann geläufigen Weise erfolgen.

In Präparaten, in denen die erfindungsgemässen Verbindungen in Kombination mit Sulfonamiden verabreicht werden, kann das Gewichtsverhältnis der beiden Komponenten zueinander innerhalb weiter Grenzen variieren. Es kann zwischen 1:40 und 10:1 liegen und beträgt vorzugsweise 1:5 bis 5:1. Eine Tablette kann beispielsweise 80 bis 400 mg einer Verbindung I und 400—80 mg eines Sulfonamids enthalten. Bei Präparaten mit einer Verbindung I als einziger aktiver Komponente kann als Richtwert für eine Einzeldosis 100—1000 mg gelten, die, je nach Erfordernis, ein- oder mehrmals täglich verabreicht werden kann.

Beispiel 1

Eine Lösung von 36 g 3,5-Dimethoxy-4-methylthiobenzaldehyd und 28 g β-Morpholinopropionitril in 300 ml Dimethylsuloxid wurde auf 60°C erwärmt und mit 3,9 g festem Natriummethylat versetzt. Unter leicht exothermer Reaktion verfärbte sich die Lösung sofort dunkel. Sie wurde im Oelbad (60°C) eine Stunde gerührt, abgekühlt und in Eiswasser gegossen. Dann wurde mit Essigester extrahiert. mit Kochsalzlösung gewaschen und über Natriumsulfat getrocknet. Nach dem Entfernen des Lösungsmittels erhielt man 56 g (99%) 3,5-Dimethoxy-α-(morpholinomethylen)-4-(methylthio)-hydrozimtsäurenitril in Form eines hellgelben Oels (Smp. 110—113°C, aus Essigester/Pentan), das in dieser Form wieterverarbeitet wurde.

Zu einem unter Eiskühlung hergestellten Gemisch aus 18,6 g Anilin und 11,5 g Essigsäure wurden, 56 g, 3,5-Dimethoxy-α-(morpholinomethylen)-4-(methylthio)-hydrozimtsäurenitril, gelöst in 250 ml Isopropanol, gegeben. Man erwärmte 2 Stunden unter Rückfluss, destillierte gegen Ende der Reaktion 100 ml Isopropanol ab, kühlte auf 0°C ab, versetzte mit 300 ml Wasser und extrahierte mit Essigester. Die organische Phase wurde mit Kochsalzlösung gewaschen, über Natriumsulfat getrocknet und eingeent. Aus dem Rückstand kristallisierten 50 g rohes α-Anilinomethylen-3,5-dimethoxy-4-(methylthio)-hydrozimtsäurenitril. Umkristallisation aus Essigester/Pentan lieferte 40 g (72%) der Verbindung mit einem Schmelzpunkt von 206—210°C.

Eine Lösung von 10,2 g $\alpha$-anilinomethylen-3,5-dimethoxy-4-(methylthio)-hydrozimtsäurenitril und 16,6 g Guanidincarbonat in 200 ml Aethanol, enthaltend 2,15 g Natrium, wurde 20 Stunden unter Rückfluss erhitzt. Das Lösungsmittel wurde abgezogen, der Rückstand in Eiswasser aufgeschlämmt und die Kristalle abgenutscht, erschöpfend mit Eiswasser gewaschen und aus heissem Aethanol umkristallisiert. Es wurden 7,1 g (77,5%) 2,4-Diamino-5-[3,5-dimethoxy-4-(methylthio)-benzyl]-pyrimidin, Smp. 231—232°C, erhalten.

Beispiel 2

In Analogie zu der in Beispiel 1 beschriebenen Methode wurden weitere Aldehyde der Formel VIII über Verbindungen II-c und II-d in Verbindungen der Formel I übergeführt. Ergebnisse siehe Tabelle 2.

TABELLE 2

| $R^1$ | $R^2$ | $R^3$ | $R^4$ | Verb. III [g] | Verb. II-c Ausbeute [g] | Verb. II-c Ausbeute [%] | Verb. II-c Smp. [°C] | Verb. II-c [g] | Verb. II-d Ausbeute [g] | Verb. II-d Ausbeute [%] | Verb. II-d Smp. [°C] | Verb. II-d [g] | Verb. I Ausbeute [g] | Verb. I Ausbeute [%] | Verb. I Smp. [°C] | Bemerkungen |
|---|---|---|---|---|---|---|---|---|---|---|---|---|---|---|---|---|
| H | $OCH_3$ | $SC_2H_5$ | $OCH_3$ | 5,3 | 5,3 | 57 | 125–130 | 5 | 4,3 | 86 | 117–119 | 8,3 | 4,7 | 62 | 201–203 | 1) ohne Reinigung weiterverarbeitet |
| H | $SCH_3$ | 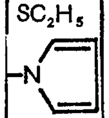 | $SCH_3$ | 19,6 | 22 | 77 | 152–153 | 8,5 | 8,0 | 94 | 167–169 | 8,3 | 4,5 | 59,5 | 189–191 | |
| $CH_3$ | H | $SCH_3$ | $SCH_3$ | 7,73 | 10,3 | 84 | 94–96 | 10,3 | 9,0 | 87 | – | 9,0 | 5,1 | 63 | 224–225 | |
| H | H | $SCH_3$ | $SCH_3$ | 2,2 | 3,56 | 71 | 86–88 | 3,6 | 3,7 2) | >90 | 136–140 | 7,2 | 3,75 | 55 | 163–164 | 2) Rohprodukt |
| H | $SCH_3$ | $OCH_3$ | $SCH_3$ | 2,6 | 3,9 | 90 1) | Oel | 3,9 | 2,9 | 73 | 135–139 | 5,4 | 2,6 | 50 | 192–193 | |
| H | $SCH_3$ | $SCH_3$ | $SCH_3$ | 2,4 | 3,1 | 85 | 143 | 4,7 | 3,4 | 71 | 171–173 | 4,9 | 2,1 | 54 | 222–224 | |

Beispiel 3

Ein Gemisch aus 10,6 g 3,5-Dimethoxy-4-methylthiobenzaldehyd, 6,2 g Cyanessigsäureäthylester und 2 Tropfen Piperidin wurde im offenen Kolben unter Rühren 90 Minuten auf 120°C erwärmt, dann abgekühlt und aus Aether/n-Heptan umkristallisiert. Die Ausbeute an $\alpha$-Cyano-3,5-dimethoxy-4-methylthiozimtsäureäthylester, Smp. 98—100°C (aus Aethanol), betrug 12,0 g (80%).

Zu einer Suspension von 6,0 g $\alpha$-Cyano-3,5-dimethoxy-4-methylthio-zimtsäureäthylester in 130 ml Aethanol und 2 Tropfen 1N Natronlauge wurden unter Rühren 0,23 g Natriumborhydrid gegeben, wobei eine Lösung entstand. Nach 30 Minuten wurde unter Wasserstrahlvakuum bei 40°C das Aethanol abgedampft, der Rückstand in Aether aufgenommen, die Lösung mit Wasser gewaschen und durch Kieselgel filtriert. Es wurden 5,0 g (79,1%) $\alpha$-Cyano-3,5-dimethoxy-4-methylthio-hydrozimtsäureäthylester als farbloses Oel erhalten.

In einem anderen Versuch wurde eine Lösung von 3 g $\alpha$-Cyano-3,5-dimethoxy-4-methylthio-zimtsäureäthylester in 250 ml Methanol und 500 mg 5% Pd/C 24 Stunden unter Normaldruck und bei Raumtemperatur hydriert. Nach üblicher Aufarbeitung wurden 3,0 g (~100%) $\alpha$-Cyano-3,5-dimethoxy-4-methylthio-hydrozimtsäureäthylester isoliert.

Zu einer Lösung von 0,7 g Natrium in 60 ml abs. Aethanol wurden 2,7 g Guanidinhydrochlorid gegeben. Nach 30 Minuten wurde die Suspension filtrierte und zum Filtrat 4,5 g $\alpha$-Cyano-3,5-dimethoxy-4-methylthio-hydrozimtsäureäthylester gegeben. Das Reaktionsgemisch wurde 18 Stunden unter Rückfluss gekocht, zur Trockene eingedampft, der Rückstand mit Wasser aufgeschlemmt und dreimal mit 200 ml Essigester extrahiert. Der Extrakt wurde eingedampft und der Rückstand aus Methanol kristallisiert. Es wurden 1,8 g (38,5%) 2,6-Diamino-5-(3,5-dimethoxy-4-methylthiobenzyl)-4-pyrimidinol, Smp. 213—215°C, erhalten.

Zu einer Suspension von 0,96 g 2,6-Diamino-5-(3,5-dimethoxy-4-methylthio-benzyl)-4-pyrimidinol in 7,6 g Phosphoroxychlorid wurden unter Rühren tropfenweise 0,73 g Dimethylanilin gegeben. Die Mischung wurde 4 Stunden unter Rühren gekocht und ungefähr die Hälfte des überschüssigen Phosphoroxychlorids wurde unter vermindertem Druck abdestilliert. Zum Rest werden ca. 10 g Eis gegeben. Die Suspension wurde dann bei Raumtemperatur 36 Stunden stehengelassen und mit konz. Ammoniaklösung auf pH 10 eingestellt. Die entstandene Suspension wurde durch eine Wasserdampfdestillation von Dimethylanilin befreit. Nach dem Erkalten der wässrigen Suspension wurde das feste Rohprodukt abgenutscht, in Benzol/Methanol (4:1, v/v) gelöst und durch Kieselgel filtriert. Nach Abdampfen des Lösungsmittels wurde das erhaltene 2,6-Diamino-4-chlor-5-(3,5-dimethoxy-4-methylthiobenzyl)-pyrimidin aus Methanol umkristallisiert; Smp. 218—221°C.

Zu einer Lösung von 167,7 mg 2,6-Diamino-4-chlor-5-(3,5-dimethoxy-4-methylthiobenzyl)-pyrimdin in 1,6 ml Eisessig und 10 mg Quecksilber(II)-chlorid in 0,2 ml Wasser wurden 150 mg Zinkpulver gegeben. Das Gemisch wurde über Nacht unter Rühren und Rückfluss gekocht. Die erhaltene Lösung wurde heiss filtriert, wobei nicht umgesetztes Zink mit 0,5 ml Eisessig nachgewaschen wurde. Das Filtrat wurde mit 2 ml Wasser verdünnt und unter Kühlung mit konz. Ammoniaklösung alkalisch gestellt. Die Suspension wurde dreimal mit 5 ml Essigester extrahiert, der Extrakt getrocknet und eingeengt und der Rückstand aus Methanol umkristallisiert. Es wurden 148 mg 2,4-Diamino-5-(3,5-dimethoxy-4-methylthio-benzyl)-pyrimidin, Smp. 231—232°C, erhalten.

Beispiel 4

Ein Gemisch aus 3,03 g 3,4-Bis-(methylthio)-toluol, 2,8 g 2,4-Diamino-5-methoxymethyl pyrimidin und 15 ml Orthophosphorsäure wurde 6 Stunden bei 100° gerührt. Nach Abkühlung und Verdünnung mit 5 ml Wasser wurden 45 ml einer 50%igen Kaliumhydroxidlösung bei 0° zugetropft. Das feste Produkt wurde abgenutscht, zweimal mit Wasser verrührt, in Methanol gelöst und das Lösungsmittel abgedampft. Der Rückstand (2,5 g) wurde durch Säulenchromatographie an Kieselgel mit Essigester/Methanol (3:1 v/v) als Elutionsmittel gereinigt. Das isolierte 2,4-Diamino-5-[2-methyl-4,5-bis-(methylthio)-benzyl]-pyrimidin wurde aus Methanol umkristallisiert; Smp. 217—222°C.

Beispiel 5

Eine Lösung von 20,7 g Natrium in 250 ml Methanol wurde mit 86 g Guanidin-hydrochlorid versetzt. Nach Filtration wurde die Lösung innerhalb von 30 Minuten zu einer Lösung von 98 g frisch hergestelltem 3,5-Dimethoxy-4-(methylthio)-$\alpha$-(2-methoxyäthoxymethylen)-hydrozimtsäurenitril in 250 ml Aethylenglykolmonomethyläther bei 95°C zugetropft, wobei das Methanol laufend abdestilliert wurde. Die Temperatur wurde 1 Stunde auf 120°C erhöht und während dieser Zeit wurden 200 ml Lösungsmittel unter vermindertem Druck abdestilliert. Das Reaktionsgemisch wurde dan auf 0°C abgekühlt und mit 400 ml Wasser versetzt. Der kristalline Niederschlag wurde abgenutscht und nacheinander mit Wasser, Aethanol und Aether gewaschen. Ausbeute: 59 g farbloses 2,4-Diamino-5-[3,5-dimethoxy-4-(methylthio)-benzyl]-pyrimidin, Smp. 219—223°C.

Das Ausgangsmaterial wurde folgendermassen hergestellt:

Zu einer Lösung von 3,5 g Natrium in 500 ml wasserfreim Aethylenglykolmonomethyläther wurden tropfenweise 71 g $\beta$-Methoxy-äthoxypropionitril gegeben. Die Lösung wurde auf 90°C erwärmt und unter Rühren portionsweise mit 106 g 3,5-Dimethoxy-4-(methylthio)-benzaldehyd versetzt. Das Reaktionsgemisch wurde auf 125°C erwärmt, wobei 50 ml Lösungsmittel abdestillierten.

Restliches Lösungsmittel wurde unter vermindertem Druck bei 65°C entfernt, der Rückstand in Aethylenchlorid aufgenommen und 3 x mit je 300 ml Wasser gewaschen. Die wässrigen Phasen wurden noch zweimal mit 300 ml Aethylenchlorid extrahiert, die vereinigten organischen Phasen über Natriumsulfat getrocknet und zur Trockene eingeengt. Das erhaltene dunkle Oel (155 g) wurde unter Hochvakuum beit 200°C destilliert und lieferte 98 g (60%) 3,5-Dimethoxy-4-(methylthio)-$\alpha$-(2-methoxyäthoxymethylen)-hydrozimtsäurenitril in Form eines zähflüssigen gelben Oels, das sofort weiterverarbeitet wurde.

Beispiel 6

Eine Lösung von 5,3 g 3,5-Dimethoxy-4-(methylthio)-benzaldhyd und 3,1 g $\beta$-Imidazolylpropionitril in 50 ml Aethanol wurde mit 1,35 g Natriummethylat versetzt und dann 5 Stunden unter Rückfluss erhitzt. Der braunen Lösung wurde eine Suspension von 4,8 g Guanidinhydrochlorid und 2,7 g Natriummethylat in 50 ml Aethanol zugesetzt. Aethanol wurde abdestilliert und der Rückstand 90 Minuten bei 100°C nachgerührt. Das Reaktionsgemisch wurde in Wasser aufgeschlämmt und filtriert, das Filtrat eingeengt und der Rückstand aus Aethanol/Aether umkristallisiert. Es wurden 3,6 g (48%) 2,4-Diamino-5-[3,5-dimethoxy-4-(methylthio)-benzyl]-pyrimidin, Smp. 224—227°C, in Form hellgelber Kristalle erhalten.

Beispiel 7

Herstellung von Ausgangsmaterialien:

(A) Zu einer auf 0—5°C gehaltenen Suspension von 63,3 g 4-Amino-3,5-dimethoxybenzoesäuremethylester in 300 ml Wasser und 75 ml konzentrierter Salzsäure wurde eine Lösung von 23 g Natriumnitrit in 60 ml Wasser getropft. Die nun klare orangefarbene Lösung wurde in eine eiskalte, kräftig gerührte Lösung von 80 g Kaliummethylxanthogenat in 300 ml Wasser getropft, wobei starke Gasentwicklung zu beobachten war. Nach vierstündigem Rühren bei Raumtemperatur wurde mit Essigester extrahiert, der Extrakt mit Kochsalzlösung gewaschen und über Natriumsulfat getrocknet. Aus dem nach dem Einengen verbleibenden Rückstand wurden 54 g (60%) rohes S-[4-(Methoxycarbonyl)-2,6-dimethoxyphenyl]-O-methyldithiocarbonat, Smp. 113—115°C, erhalten.

Ein Gemisch aus 29 g S-[4-(Methoxycarbonyl)-2,6-dimethoxyphenyl]-O-methyldithiocarbonat und 2 g Kaliummethylxanthogenat wurden in 450 ml Dimethylformamid 1 Stunde bei 100°C gerührt. Dann wurde das Lösungsmittel unter vermindertem Druck entfernt und der Rückstand in Essigester aufgenommen. Nach Waschen mit Wasser und gesättigter Kochsalzlösung wurde öber Natriumsulfat getrocknet und das Lösungsmittel abgezogen. Der Rückstand lieferte nach Umkristallisation aus Cyclohexan 18,8 g (81%) 3,5-Dimethoxy-4-(methylthio)-benzoesäuremethylester in Form farbloser Kristalle, Smp. 86—88°C.

Zu einer auf 0°C gekühlten Lösung von 150 g Natriumdimethoxyäthoxyalanat (70% in Toluol) in 150 ml Toluol wurden 50 g Morpholin in 200 ml Toluol getropft. Die klare Lösung wurde bei 0° nachgerührt und dann tropfenweise unter Rühren zu einer auf −20°C gekühlten Lösung von 30 g 3,5-Dimethoxy-4-(methylthio)-benzoesäuremethylester in 700 ml Toluol gegeben. Es wurde noch 5 Stunden gerührt. Anschliessend wurden langsam 100 ml Wasser zugetropft, 100 ml 3N Salzsäure zugegeben und die Phasen im Scheidetrichter getrennt. Die wässrige Phase wurde zweimal mit Essigester extrahiert, während die organischen Phasen mit Kochsalzlösung gewaschen und über Natriumsulfat getrocknet wurden. Einengen der Lösung ergab 26 g eines gelben Oeles, das durchkristallisierte und nach Umkristallisation aus Essigester/Pentan 21 g (78%) 3,5-Dimethoxy-4-(methylthio)-benzaldehyd, Smp. 54—56°C, lieferte.

(B) In zu (A) analoger Weise wurden 21,1 g 4-Amino-3,5-dimethoxybenzoesäuremethylester in 19 g (68%) S-[4-Methoxycarbonyl)-2,6-dimethoxyphenyl]-O-äthyldithiocarbonat, Smp. 84—86°C, übergeführt und dieses in 90%iger Ausbeute weiter zu 3,5-Dimethoxy-4-äthylthiobenzoesäuremethylester, Smp. 50—52°C, umgesetzt.

Zu einer bei 0°C gerührten Lösung von 10,8 g 3,5-Dimethoxy-4-äthylthio)-benzoesäuremethylester in 100 ml absolutem Aether wurden innert 10 Minuten 120 ml einer 20%igen Diisobutylaluminiumhydridlösung in Hexan getropft. Es wurde während 2 Stunden bei 0—5°C weitergerührt, nach langsamen Zutropfen von 100 ml Wasser mit 150 ml 3N Salzsäure versetzt, in Aether aufgenommen und neutralgewaschen. Die Lösung wurde über Natriumsulfat getrocknet, das Lösungsmittel abgezogen, das verbleibende gelbe Oel (11,2 g) in 100 ml Methylenchlorid aufgenommen und die Lösung in Gegenwart von 15 g Mangandioxid bei Raumtemperatur gerührt. Nach 3 Stunden wurden nochmals 15 g Mangandioxid zugegeben. Dann wurde 3 Stunden gerührt, durch ein Filtrierhilfsmittel auf der Basis von Diatomeenerde filtriert und das Filtrat eingeengt. Der Rückstand, aus Aether/Pentan kristallisiert, lieferte 9,2 g (96,5%) 3,5-Dimethoxy-4-(äthylthio)-benzaldehyd in Form farbloser Kristalle vom Smp. 38—39°C.

(C) Zu einer Suspension von Natriummercaptid, hergestellt aus 20,7 g 55%iger Natriumhydrid-Dispersion (in Mineralöl) in 160 ml Dimethylformamid und 27 ml Methylmercaptan in 110 ml Dimethylformamid, wurden bei 15—25°C, unter Stickstoffbegasung, Rühren und Eiswasserkühlung portionsweise 28 g 4-Nitro-3,5-dibrombenzoesäureäthylester gegeben. Das Reaktionsgemisch wurde 4 Stunden bei Zimmertemperatur gerührt, unter Hochvakuum bei 45°C zur Trockene eingedampft, der

Rückstand mit 750 ml Wasser versetzt und 5 x mit je 150 ml Aether extrahiert. Der Aetherextrakt wurde getrocknet, filtriert und eingedampft. Aus dem Rückstand wurden nach Umkristallisation aus heissem n-Heptan 16,5 g (75%) 3,4,5-Tris-(methylthio)-benzoesäureäthylester, Smp. 90—92°C, erhalten.

Der Ester wurde in zu (B) analoger Weite in 58%iger Ausbeute zu 3,4,5-Tris-(methylthio)-bentaldehyd, Smp. 160—161°C, reduziert.

(D) Ein Gemisch aus 22 g 4-Amino-3,5-dibrombenzoesäureäthylester, 100 ml Essigsäure und 14,4 g 2,5-Diäthoxytetrahydrofuran wurde während 1 Stunde bei 100°C gehalten und dann unter vermindertem Druck eingeengt. Der dunkle Rückstand wurde in Essigester aufgenommen, die Lösung zunächst mit Natriumbicarbonatlösung, dann mit Kochsalzlösung gewaschen, über Natriumsulfat getrocknet und zur Trockene eingeengt. Aus Aether/Petroläther wurden 20 g (60%) 3,5-Dibrom-4-pyrrolo-benzoesäureäthylester, Smp. 106—107°C erhalten. 18,5 g dieser Verbindung in 40 ml warmen Dimethylformamid wurden zu einer bei Raumtemperatur gerührten Lösung von Methylmercaptid (hergestellt aus 6 g Natriumhydrid und ca. 15 g Methylmercaptan in Dimethylformamid) in 100 ml Dimethylformamid getropft. Es wurde 16 Stunden bei Raumtemperatur nachgerührt, eingeengt, in Essigester aufgenommen, mit Salzsäure auf pH 2 eingestellt, mit gesättigter Kochsalzlösung gewaschen, über Natriumsulfat getrocknet und nochmals eingeengt. Der Rückstand wurde in Aethanol/HCl verestert. Die Lösung wurde eingeengt. Aus dem Rückstand, gelöst in Aether, erhielt man nach Zugabe von Hexan 8,8 g (57%) kristallinen 3,5-Bis-(methylthio)-4-pyrrolo-benzoesäureäthylester, Smp. 108—109°C.

In zu (B) analoger Weise wurden dann 21 g 3,5-Bis-(methylthio)-4-pyrrolo-benzoesäureäthylester in 67%iger Ausbeute zu 3,5-Bis-(methylthio)-4-pyrrolo-benzaldehyd, Smp. 146—148°C, reduziert.

(E) Zu einer bei 0°C gerührten Suspension von 4,7 g Natriumhydrid (50%), das durch zweimaliges Waschen mit Pentan vom Oel befreit wurde, in 100 ml Dimethylformamid wurden tropfenweise 6,2 ml (5,0 g) Methylmercaptan zugegeben. Nach 15 Minuten wurden 12,25 g 3,5-Dimethoxy-4-brombenzaldehyd in 50 ml Dimethylformamid zugetropft. Die braune, sich aufhellende Lösung wurde bei Raumtemperatur eine halb Stunde gerührt, auf Eiswasser gegossen und sas Gemisch mit Essigester extrahiert. Der Extrakt wurde mit Wasser, kalter 1N Salzsäure und gesättigter Kochsalzlösung gewaschen, über Natriumsulfat getrocknet und das nach dem Abziehen des Lösungsmittels verbleibende gelbe Oel aus Methanol kristallisiert. Ausbeute 8,9 g (84%) 3,5-Dimethoxy-4-(methylthio)-benzaldehyd, Smp. 55—56°C.

(F) Zu einer Suspension von 16,5 g Natriumhydrid (55%) in 200 ml Dimethylformamid wurde unter Rühren und Stickstoffbegasung bei 0—5°C eine Lösung von 18 g Methylmercaptan in 70 ml Dimethylformamid zugetropft. Nach 30 Minuten Rühren bei Raumtemperatur wurden 1,5 g Kupfer(I)-chlorid und anschliessend 21 g 3,5-Dibromo-4-hydroxybenzaldehyd zugegeben. Das Reaktionsgemisch wurde 68 Stunden bei 120°C gerührt, danach mit weiteren 1,5 g Kupfer(I)-chlorid versetzt und nochmals 20 Stunden bei bei 150°C gerührt. Dann wurde im Hochvakuum eingeengt, der Rückstand in 10%iger NaCl-Lösung aufgenommen und die Lösung über Diatomeenerde filtrierte. Die wässrige Phase wurde mit Aether extrahiert, unter Kühlung mit konzentrierter Salzsäure angesäuert und mit Essigester extrahiert. Die mit Wasser gewaschenen Essigester-Extrakte wurden getrocknet und eingedampft, 9 g des erhaltenen Gemisches (insgesamt 14 g) wurden in 90 ml Aceton gelöst und mit 10,7 g Kaliumcarbonat versetzt. Zu der entstandenen Suspension wurden 7,5 ml Dimethylsulfat gegeben. Das Reaktionsgemisch wurde 18 Stunden bei 50°C gerührt, abgenutscht, die anorganischen Salze wurden mit Aceton gewaschen und das Filtrat eingedampft. Der Eindampfrückstand wurde in Essigester gelöst, sukzessiv mit konzentriertem Ammoniak und Wasser gewaschen und mit Heptan/Aceton (7:1, v/v) aufgetrennt. Zunächst wurde 3-Brom-5-methylthio-anisaldehyd, Smp. 54—56°C, Ausbeute 3,5 g, dann wurde 3,5-Bis-(methylthio)-anisaldehyd, Smp. 84—86°C (aus n-Heptan), Ausbeute 2,6 g eluiert.

(G) Zu einer Lösung von 23 g 85%igem Kaliumhydroxid in 190 ml Aethanol wurden 27,3 g 1-Methyl-3,4-dithiophenol (hergestellt aus 4-Methylanthranilsäure nach Synthesis *1976,* 471) in 110 ml Aethanol innerhalb von 20 Minuten zugetropft. Die Lösung wurde nach 45 Minuten mit 49,6 g Methyljodid in 70 ml Aethanol versetzt, das nach 30 Minuten Rühren ausgefallene KJ abfiltriert, das Aethanol abgedampft und das erhaltene 3,4-Bis-(methylthio)-toluol bei 95—98°C/1 Torr destilliert. Ausbeute 30,0 g (93%), Smp. 38—39°C (Heptan).

Zu einer Lösung von 7 g 3,4-Bis-(methylthio)-toluol in 20 g N-Methylformanilid wurden bei 15—20°C 21,6 g Phosphoroxychlorid getropft und die Lösung wurde 24 Stunden bei 55°C gerührt. Das Reaktionsgemisch wurde nochmals mit einer Suspension aus 9,6 g N-Methylformanilid und 10,8 g Phosphoroxychlorid versetzt und weitere 24 Stunden bei 55°C gerührt. Die abgekühlte Mischung wurde mit Wasser versetzt, mit Aether extrahiert, die vereinigten Extrakte wurden getrocknet, zur Trockene eingedampft und der Rückstand an Kieselgel mit Benzol chromatographiert. Es wurden 14,7 g 3,4-Bis-(methylthio)-6-methylbenzaldehyd, Smp. 51—52°C (Heptan), erhalten.

In analoger Weise wurde aus 1,2-Dithiophenol in 83%iger Ausbeute 1,2-Bis-(methylthio)-benzol erhalten, das in 39%iger Ausbeute in 3,4-Bis-(methylthio)-benzaldehyd übergeführt werden konnte; Smp. 50°C.

Beispiel 8

Es wurden Tabletten folgender Zusammensetzung hergestellt:

| | mg/Tabl. |
|---|---|
| 2,4-Diamino-5-]3,5-dimethoxy-4-(methylthio)-benzyl]-pyrimidin | 50,00 |
| Stärke kalt quellend | 12,50 |
| Natrium-carboxymethyl-stärke | 12,50 |
| Lactose pulv. | 143,75 |
| Magnesiumstearat | 1,25 |
| | 220,00 |

Das Gemisch wurde unter Zusatz von Wasser nass granuliert und zu Tabletten gepresst.

**Patentansprüche**

1. 2,4-Diamino-5-benzylpyrimidine der Formel

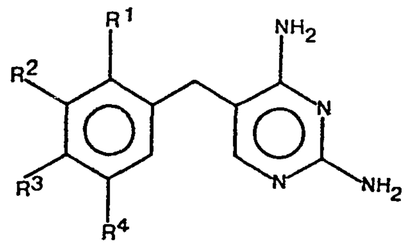

I

worin
$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen.
und deren physiologisch verträgliche Salze.

2. 2,4-Diamino-5-benzylpyrimidine der Formel

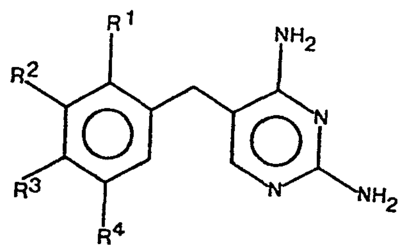

I

worin
$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ und $R^4$ $C_{1-6}$-Alkoxy und $R^3$ Carboxy-$C_{1-6}$-alkylthio oder Cyano-$C_{1-6}$-alkylthio darstellen,
und deren physiologisch verträgliche Salze

3. 2,4-Diamino-5-benzylpyrimidine der Formel

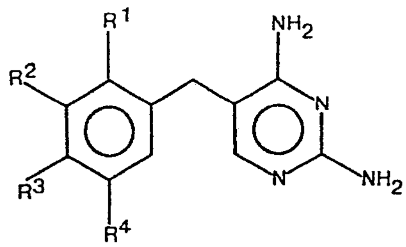

I

worin

$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ und $R^4$ $C_{1-6}$-Alkoxy und $R^3$ $C_{1-6}$-Alkylthio oder Carboxy-$C_{1-6}$-alkylthio darstellen,
und deren physiologisch verträgliche Salze.

4. 2,4-Diamino-5-benzylpyrimidine der Formel

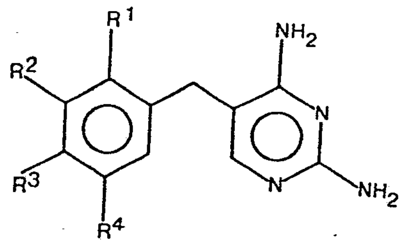

I

worin

$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ und $R^4$ $C_{1-6}$-Alkoxy und $R^3$ Carboxy-$C_{1-6}$-alkylthio darstellen,
und deren physiologisch verträgliche Salze.

5. 2,4-Diamino-5-benzylpyrimidine der Formel

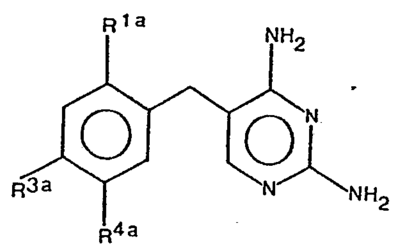

I-1

worin

$R^{1a}$ Wasserstoff oder $C_{1-6}$-Alkyl; $R^{3a}$ $C_{1-6}$-Alkylthio und $R^{4a}$ $C_{1-6}$-Alkylthio bedeuten,
und deren physiologisch verträgliche Salze.

6. 2,4-Diamino-5-benzylpyrimidine der Formel

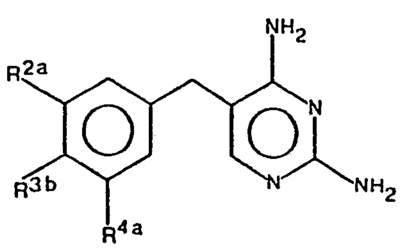

I-2

worin

$R^{2a}$ $C_{1-6}$-Alkylthio;
$R^{3b}$ $C_{1-6}$-Alkoxy, $C_{1-6}$-Alkylthio oder Pyrrolo und
$R^{4a}$ $C_{1-6}$-Alkylthio bedeuten,
und deren physiologisch verträgliche Salze.

7. 2,4-Diamino-5-benzylpyrimidine der Formel

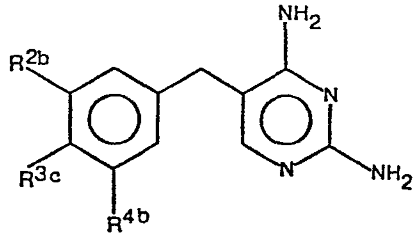

I-3

worin

$R^{2b}$ und $R^{4b}$ $C_{1-6}$-Alkoxy und

$R^{3c}$ $C_{1-6}$-Alkylthio, Carboxy-$C_{1-6}$-alkylthio oder Cyano-$C_{1-6}$-alkylthio bedeuten,

und deren physiologisch verträgliche Salze.

8. 2,4-Diamino-5-benzylpyrimidine der Formel

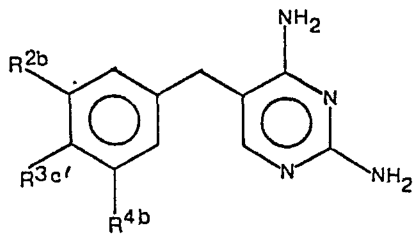

I-3'

worin

$R^{2b}$ und $R^{4b}$ und $C_{1-6}$-Alkoxy und

$R^{3c'}$ Carboxy-$C_{1-6}$-alkylthio oder Cyano-$C_{1-6}$-alkylthio bedeuten,

und deren physiologisch verträgliche Salze.

9. 2,4-Diamino-5-benzylpyrimidine der Formel

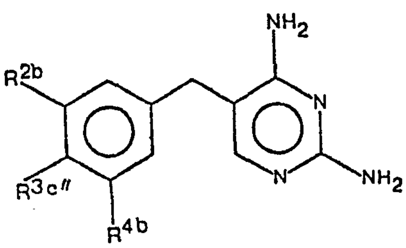

I-3''

worin

$R^{2b}$ und $R^{4b}$ $C_{1-6}$-Alkoxy und

$R^{3c''}$ $C_{1-6}$-Alkylthio oder Carboxy-$C_{1-6}$-alkylthio bedeuten,

und deren physiologisch verträgliche Salze.

10. 2,4-Diamino-5-benzylpyrimidine der Formel

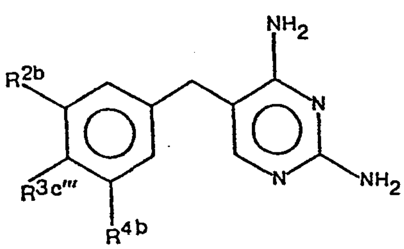

I-3'''

worin

$R^{2b}$ und $R^{4b}$ $C_{1-6}$-Alkoxy und

$R^{3c'''}$ Carboxy-$C_{1-6}$-alkylthio bedeuten,

und deren physiologisch verträgliche Salze.

11. 2,4-Diamino-5-[3,5-dimethoxy-4-(methylthio)-benzyl]-pyrimidin.

12. 2,4-Diamino-5-[4-(äthylthio)-3,5-dimethoxybenzyl]-pyrimidin.

13. 2,4-Diamino-5-[3,5-bis-(methylthio)-4-pyrrolobenzyl]-pyrimidin.

14. 2,4-Diamino-5-[3,4-bis-(methylthio)-benzyl]-pyrimidin.
15. 2,4-Diamino-5-[3,4,5-tris-(methylthio)-benzyl]-pyrimidin.
16. 2,4-Diamino-5-[2-methyl-4,5-bis-(methylthio)-benzyl]-pyrimidin.
17. 2,4-Diamino-5-[4-methoxy-3,5-bis-(methylthio)-benzyl]-pyrimidin.
18. 2,4-Diamino-5-[4-(4-cyanobutylthio)-3,5-dimethoxybenzyl]-pyrimidin.
19. 2,4-Diamino-5-[4-(carboxymethylthio)-3,5-dimethoxybenzyl]-pyrimidin.
20. Verfahren zur Herstellung von Verbindungen gemäss Anspruch 1 oder 2, dadurch gekennzeichnet, dass man
(a) eine Verbindung der Formel

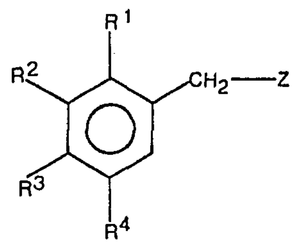

II

worin
$R^1$, $R^2$, $R^3$ und $R^4$ die in Anspruch bzw. 2 angegebenen Bedeutungen haben, Z eine Gruppe —C(CN) = CH—Y oder —CH(CN)CH$(OR^5)_2$, Y eine Abgangsgruppe und $R^5$ $C_{1-6}$-Alkyl, 2-$C_{1-6}$-Alkoxyäthyl, 2-Phenoxy-äthyl oder 2-Benzyloxy-äthyl oder beide Reste $R^5$ gemeinsam $C_{1-6}$-Alkylen darstellen,
mit Guanidin oder einem Guanidinsalz kondensiert oder
(b) aus einer Verbindung der Formel

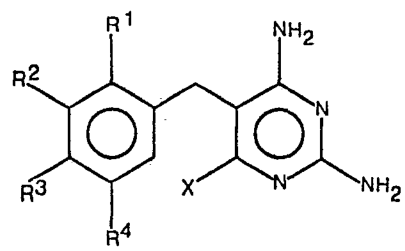

III

worin
X Halogen darstellt und $R^1$, $R^2$, $R^3$ und $R^4$ die in Anspruch bzw. 2 angegebenen Bedeutungen haben,
den Substituenten X reduktiv eliminiert oder
(c) ein substituiertes Benzylpyrimidin der Formel

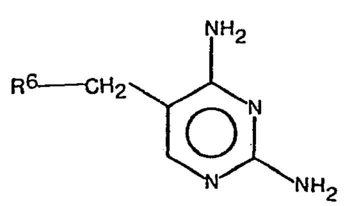

IV

worin
$R^6$ $C_{1-6}$-Alkoxy (vorzugsweise Methoxy), Benzyloxy, Hydroxy oder Halogen darstellt,
mit einer Verbindung der Formel

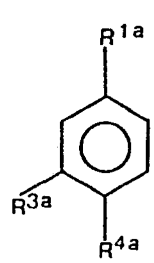

V

worin

$R^{1a}$, $R^{3a}$ und $R^{4a}$ die in Anspruch 5 angegebenen Bedeutungen haben,
umsetzt und die erhaltene Verbindung gewünschtenfalls in ein physiologisch verträgliches Salz überführt.

21. Verfahren zur Herstellung von Verbindungen gemäss Anspruch 3 oder 4, dadurch gekennzeichnet, dass man
(a) eine Verbindung der Formel

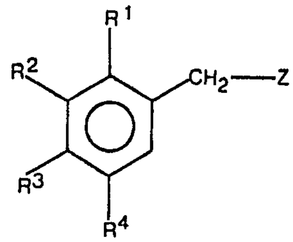

II

worin
$R^1$, $R^2$, $R^3$ und $R^4$ die in Anspruch 3 bzw. 4 angegebenen Bedeutungen haben, Z eine Gruppe $—C(CN) = CH—Y$ oder $—CH(CN)CH(OR^5)_2$;
Y eine Abgangsgruppe und $R^5$ $C_{1-6}$-Alkyl oder beide Reste $R^5$ gemeinsam $C_{1-6}$-Alkylen darstellen,
mit Guanidin oder einem Guanidinsalz kondensiert oder
(b) aus einer Verbindung der Formel

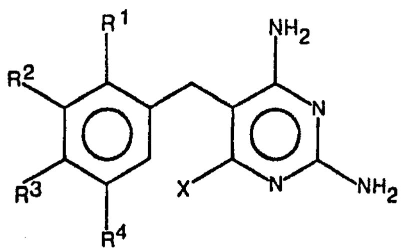

III

worin
X Halogen darstellt und $R^1$, $R^2$, $R^3$ und $R^4$ die in Anspruch 3 bzw. 4 angegebenen Bedeutungen haben,
den Substituenten X reduktiv eliminiert oder
(c) ein substituiertes Benzylpyrimidin der Formel

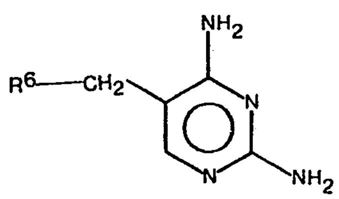

IV

worin
$R^6$ $C_{1-6}$-Alkoxy (vorzugsweise Methoxy), Benzyloxy, Hydroxy oder Halogen darstellt,
mit einer Verbindung der Formel

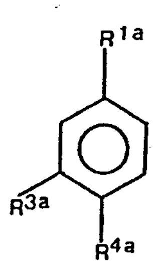

V

worin
$R^{1a}$, $R^{3a}$ und $R^{4a}$ die in Anspruch 5 angegebenen Bedeutungen haben,

umsetzt und die erhaltene Verbindung gewünschtenfalls in ein physiologisch verträgliches Salz überführt.

22. Pharmazeutische Präparate auf der Basis einer Verbindung gemäss Anspruch 1 oder 2.

23. Pharmazeutische Präparate auf der Basis einer Verbindung gemäss Anspruch 3 oder 4.

24. Pharmazeutische Präparate auf der Basis einer Verbindung gemäss Anspruch 1, oder 2 sowie eines antibakteriell wirksamen Sulfonamids.

25. Pharmazeutische Präparate auf der Basis einer Verbindung gemäss Anspruch 3 oder 4 sowie eines antibakteriell wirksamen Sulfonamids.

26. Verfahren zur Herstellung pharmazeutischer Präparate gemäss den Ansprüchen 22—25, dadurch gekennzeichnet, dass man die Wirksubstanzen mit zur therapeutischen Verabreichung geeigneten, nichttoxischen, inerten, an sich in solchen Präparaten üblichen, festen oder flüssigen Trägern vermischt und das erhaltene Gemisch in eine geeignete galenische Form bringt.

**Revendications**

1. 2,4-diamino-5-benzylpyrimidines de formule générale:

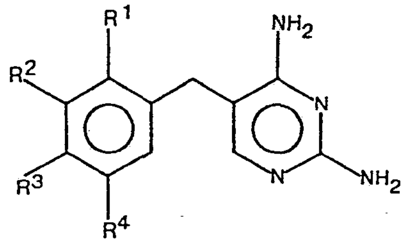

I

dans laquelle

$R^1$ représente de l'hydrogène ou un groupe alkyle en $C_1$—$C_6$, $R^2$ représente de l'hydrogène $R^3$ et $R^4$ représentent un groupe alkylthio en $C_1$—$C_6$, ou

$R^1$ représente de l'hydrogène, $R^2$ et $R^4$ représentent chacun un groupe alkylthio en $C_1$—$C_6$, $R^3$ représente un groupe alcoxy en $C_1$—$C_6$, alkylthio en $C_1$—$C_6$, ou pyrrolo, ou

$R^1$ représente de l'hydrogène, $R^2$ et $R^4$ représentent un groupe alcoxy en $C_1$—$C_6$, $R^3$ représente un groupe alkylthio en $C_1$—$C_6$, carboxyalkylthio à reste alkyle en $C_1$—$C_6$, ou cyano ($C_1$—$C_6$) alkylthio et leurs sels physiologiquement acceptables.

2. 2,4-diamino-5-benzylpyrimidines de formule générale:

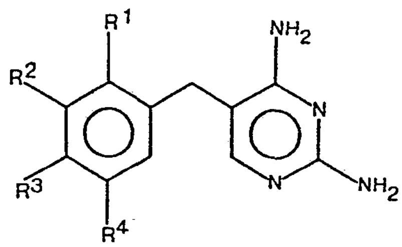

I

dans laquelle

$R^1$ représente de l'hydrogène ou un groupe alkyle en $C_1$—$C_6$, $R^2$ représente de l'hydrogène, $R^3$ et $R^4$ représentent un groupe alkylthio en $C_1$—$C_6$, ou

$R^1$ représente de l'hydrogène, $R^2$ et $R^4$ représentent chacun un groupe alkylthio en $C_1$—$C_6$, $R^3$ représente un groupe alcoxy en $C_1$—$C_6$, alkylthio en $C_1$—$C_6$, ou pyrrolo ou

$R^1$ représente de l'hydrogène; $R^2$ et $R^4$ représentent un groupe alcoxy en $C_1$—$C_6$ et $R^3$ représente un groupe carboxy ($C_1$—$C_6$)alkylthio ou cyano ($C_1$—$C_6$)alkylthio et leurs sels physiologiquement acceptables.

3. 2,4-diamino-5-benzylpyrimidines de formule générale:

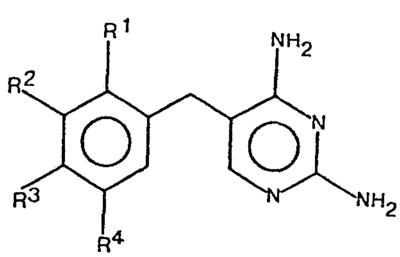

I

dans laquelle

$R^1$ représente de l'hydrogène ou un groupe alkyle en $C_1$—$C_6$,

$R^2$ représente de l'hydrogène, $R^3$ et $R^4$ représentent un groupe alkylthio en $C_1$—$C_6$, ou

$R^1$ représente de l'hydrogène, $R^2$ et $R^4$ représentent chacun un groupe alkylthio en $C_1$—$C_6$, $R^3$ représente un groupe alcoxy en $C_1$—$C_6$, alkylthio en $C_1$—$C_6$, ou pyrrolo, ou

$R^1$ représente de l'hydrogène; $R^2$ et $R^4$ $C_1$—$C_6$ alcoxy et $R^3$ représentent un groupe $C_1$—$C_6$ alkylthio ou carboxy ($C_1$—$C_6$) alkylthio et leurs sels physiologiquement acceptables.

4. 2,4-diamino-5-benzylpyrimidines de formule générale:

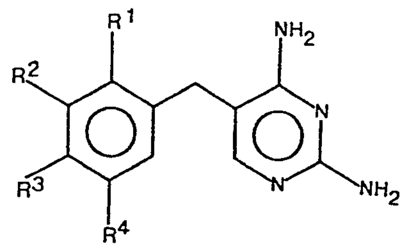

I

dans laquelle

$R^1$ représente de l'hydrogène ou un groupe alkyle en $C_1$—$C_6$,

$R^2$ représente de l'hydrogène, $R^3$ et $R^4$ représentent un groupe alkylthio en $C_1$—$C_6$, ou

$R^1$ représente de l'hydrogène, $R^2$ et $R^4$ représentent chacun un groupe alkylthio en $C_1$—$C_6$, $R^3$ représente un groupe alcoxy en $C_1$—$C_6$, alkylthio en $C_1$—$C_6$ ou pyrrolo, ou

$R^1$ représente de l'hydrogène; $R^2$ et $R^4$ représentent un groupe alcoxy en $C_1$—$C_6$ et $R^3$ représente un groupe carboxy ($C_1$—$C_6$)alkylthio et leurs sels physiologiquement acceptables.

5. 2,4-diamino-5-benzylpyrimidines de formule générale:

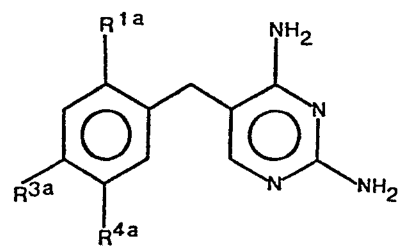

I-1

dans laquelle

$R^{1a}$ représente de l'hydrogène ou un groupe alkyle en $C_1$—$C_6$, $R^{3a}$ représente un groupe alkylthio en $C_1$—$C_6$, et $R^{4a}$ représente un groupe alkylthio en $C_1$—$C_6$, et leurs sels physiologiquement acceptables.

6. 2,4-diamino-5-benzylpyrimidines de formule générale:

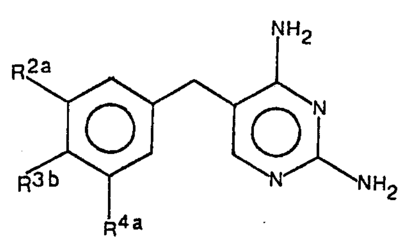

I-2

dans laquelle

$R^{2a}$ représente un groupe alkylthio en $C_1$—$C_6$, $R^{3b}$ représente un groupe alcoxy en $C_1$—$C_6$, alkylthio en $C_1$—$C_6$ ou un groupe pyrrolo et $R^{4a}$ représente un groupe alkylthio en $C_1$—$C_6$, et leurs sels physiologiquement acceptables.

7. 2,4-diamino-5-benzylpyrimidines de formule générale:

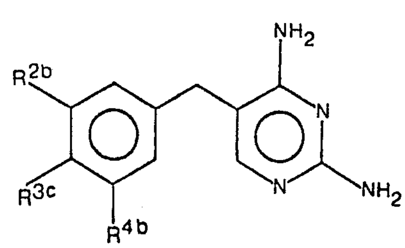

I-3

dans laquelle

$R^{2b}$ et $R^{4b}$ représentent un radical alcoxy en $C_1$ à $C_6$, $R^{3c}$ représente un groupe alkylthio en $C_1$—$C_6$, carboxy-($C_1$—$C_6$)-alkylthio ou cyano ($C_1$—$C_6$)alkylthio, et leurs sels physiologiquement acceptables.

8. 2,4-diamino-5-benzylpyrimidines de formule générale:

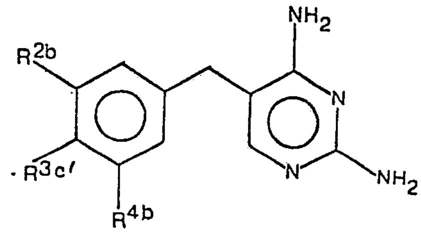

I-3'

dans laquelle

$R^{2b}$ et $R^{4b}$ représentent un groupe alcoxy en $C_1$—$C_6$, $R^{3c'}$ représente un groupe carboxyalkylthio à reste alkyle en $C_1$—$C_6$, cyanalkylthio à reste alkyle en $C_1$—$C_6$, et leurs sels physiologiquement acceptables.

9. 2,4-diamino-5-benzylpyrimidines de formule générale:

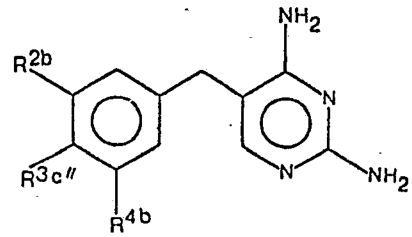

I-3''

dans laquelle

$R^{2b}$ et $R^{4b}$ représentent un groupe alcoxy en $C_1$—$C_6$, $R^{3c''}$ représente un groupe alkylthio en $C_1$—$C_6$, carboxyalkylthio à reste alkyle en $C_1$—$C_6$, et leurs sels physiologiquement acceptables.

10. 2,4-diamino-5-benzylpyrimidine de formule générale:

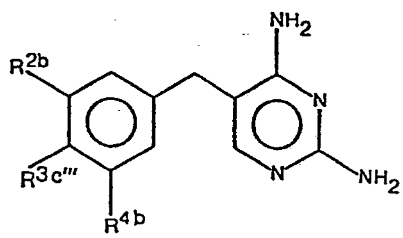

I-3'''

dans laquelle

$R^{2b}$ et $R^{4b}$ représentent un groupe alcoxy en $C_1$—$C_6$, $R^{3c'''}$ représente un groupe carboxyalkylthio à reste alkyle en $C_1$—$C_6$, et leurs sels physiologiquement acceptables.

11. La 2,4 - diamino - 5 - [3,5 - diméthoxy - 4 - (méthylthio) - benzyl] - pyrimidine.

12. La 2,4 - diamino - 5 - [4 - (éthylthio) - 3,5 - diméthoxy - benzyl] - pyrimidine.

13. La 2,4 - diamino - 5 - (3,5 - bis - (méthylthio) - 4 - pyrrolobenzyl) - pyrimidine.

14. La 2,4 - diamino - 5 - [3,4 - bis - (méthylthio) - benzyl] - pyrimidine.

15. La 2,4 - diamino - 5 - [3,4,5 - tris - (méthylthio) - benzyl] - pyrimidine.

16. La 2,4 - diamino - 5 - [2 - méthyl - 4,5 - bis - (méthylthio) - benzyl] - pyrimidine.

17. La 2,4 - diamino - 5 - [4 - méthoxy - 3,5 - bis - (méthylthio) - benzyl] - pyrimidine.

18. La 2,4 - diamino - 5 - [3,5 - diméthoxy - 4 - (4 - cyanobutylthio) - benzyl] - pyrimidine.

19. La 2,4 - diamino - 5 - (4 - carboxyméthylthio) - 3,5 - diméthoxybenzyl) - pyrimidine.

20. Procédé pour la préparation de composés selon la revendication 1 ou 2, caractérisé par le fait que:

a) on condense, avec la guanidine ou l'un de ses sels, un composé de formule:

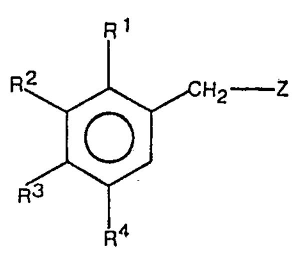

II

dans laquelle

$R^1$, $R^2$, $R^3$ et $R^4$ ont les significations données dans les revendications 1 ou 2, Z représente un groupe —C(CN)=CH—Y ou —CH(CN)CH($OR^5$)$_2$, Y représente un groupe de départ et $R^5$ représente un groupe alkyle en $C_1$—$C_6$, un groupe 2-alcoxyéthyle à reste alcoxy en $C_1$—$C_6$, un groupe 2-phénoxyéthyle ou un groupe 2-benzyloxyéthyle ou, ensemble, les deux restes $R^5$ représentent un groupe alkylène en $C_1$—$C_6$, ou

b) d'un composé formule:

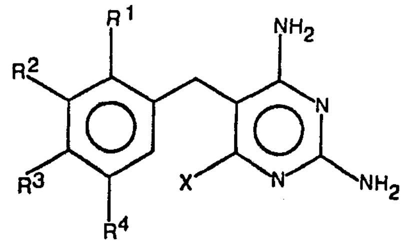

III

dans laquelle

X représente un halogène et $R^1$, $R^2$, $R^3$ et $R^4$ ont les significations données dans les revendications 1 ou 2, on élimine par réduction le substituant X ou

c) on fait réagir une benzylpyrimidine substituée de formule (IV):

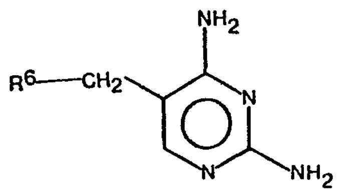

IV

dans laquelle

$R^6$ représente un groupe alcoxy en $C_1$—$C_6$ (de préférence le groupe méthoxy), le groupe benzyloxy, le groupe hydroxy ou un halogène, avec un composé de formule:

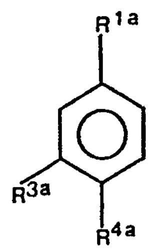

V

dans laquelle

$R^{1a}$, $R^{3a}$ et $R^{4a}$ ont les significations données dans la revendication 5, et, si on le désire, on transforme le composé ainsi obtenu en un sel physiologiquement acceptable.

21. Procédé pour la préparation de composés selon la revendication 3 ou 4, caractérisé par le fait que:

a) on condense, avec la guanidine ou l'un de ses sels, un composé de formule:

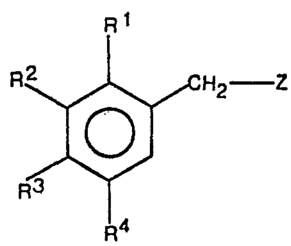

II

dans laquelle

$R^1$, $R^2$, $R^3$ et $R^4$ ont les significations donnéesd dans les revendications 3 et 4, Z représente un groupe —C(CN)=CH—Y ou —CH(CN)CH($OR^5$)$_2$ Y représente un groupe de départ et $R^5$ représente un groupe alkyle en $C_1$—$C_6$, ou ensemble, les deux restes $R^5$ représentent un groupe alkylène en $C_1$—$C_6$, ou

b) d'un composé de formule:

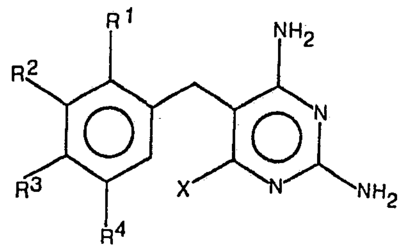

III

dans laquelle

X représente un halogène et $R^1$, $R^2$, $R^3$ et $R^4$ ont les significations données dans les revendications 3 et 4, on élimine par réduction le substituant X ou

c) on fait réagir une benzylpyrimidine substituée de formule:

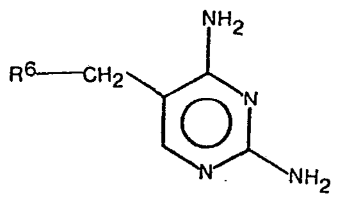

IV

dans laquelle

$R^6$ représente un groupe alcoxy en $C_1$—$C_6$ (de préférence le groupe méthoxy), le groupe benzyloxy, le groupe hydroxy ou un halogène, avec un composé de formule:

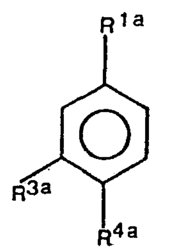

V

dans laquelle

$R^{1a}$, $R^{3a}$ et $R^{4a}$ ont les significations données dans la revendication 5, et si on le désire, on transforme le composé ainsi obtenu en un sel physiologiquement acceptable.

22. Composition pharmaceutique sur la base d'un composé selon la revendication 1 ou 2.

23. Composition pharmaceutique sur la base d'un composé selon la revendication 3 ou 4.

24. Composition pharmaceutique sur la base d'un composé selon la revendication 1 ou 2 ainsi que sur une sulfonamide anti-bactérienne.

25. Composition pharmaceutique sur la base d'un composé selon la revendication 3 ou 4 ainsi que sur une sulfonamide anti-bactérienne.

26. Procédé pour la préparation d'une composition pharmaceutique selon l'une des revendications 22 à 25, caractérisé en ce que l'on mélange les substances actives avec des supports solides ou fluides, utilisables dans de telles compositions, inertes, non toxiques et utiles en thérapeutique et en ce que l'on met en forme galénique le mélange obtenu.

**Claims**

1. 2,4-Diamino-5-benzylpyrimidines of the formula

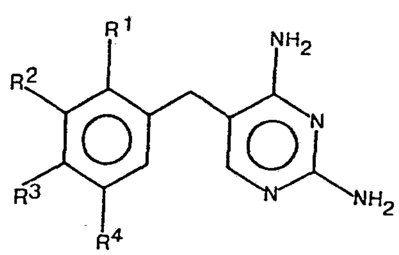

I

wherein

$R^1$ represents hydrogen or $C_{1-6}$-alkyl; $R^2$ represents hydrogen and $R^3$ and $R^4$ represent $C_{1-6}$-alkylthio or

$R^1$ represents hydrogen; $R^2$ and $R^4$ represents $C_{1-6}$-alkylthio and $R^3$ represents $C_{1-6}$-alkoxy, $C_{1-6}$-alkylthio or pyrrolo or

$R^1$ represents hydrogen; $R^2$ and $R^4$ represent $C_{1-6}$-alkoxy and $R^3$ represents $C_{1-6}$-alkylthio, carboxy-$C_{1-6}$-alkylthio or cyano-$C_{1-6}$-alkylthio,

and their physiologically compatible salts.

2. 2,4-Diamino-5-benzylpyrimidines of the formula

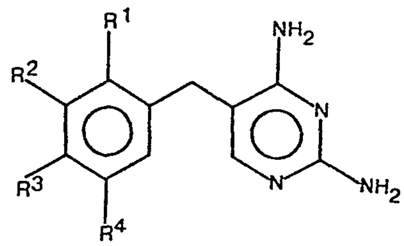

I

wherein

$R^1$ represents hydrogen or $C_{1-6}$-alkyl; $R^2$ represents hydrogen and $R^3$ and $R^4$ represent $C_{1-6}$-alkylthio or

$R^1$ represents hydrogen; $R^2$ and $R^4$ represent $C_{1-6}$-alkylthio and $R^3$ represents $C_{1-6}$-alkoxy, $C_{1-6}$-alkylthio or pyrrolo or

$R^1$ represents hydrogen; $R^2$ and $R^4$ represent $C_{1-6}$-alkoxy and $R^3$ represents carboxy $C_{1-6}$-alkylthio or cyano-$C_{1-6}$-alkylthio,

and their physiologically compatible salts.

3. 2,4-Diamino-5-benzylpyrimidines of the formula

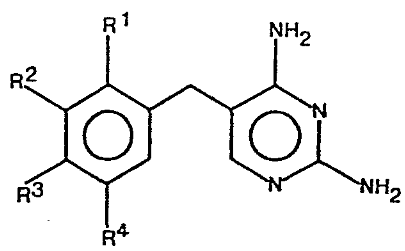

I

wherein

$R^1$ represents hydrogen or $C_{1-6}$-alkyl; $R^2$ represents hydrogen and $R^3$ and $R^4$ represents $C_{1-6}$-alkylthio or

$R^1$ represents hydrogen; $R^2$ and $R^4$ represent $C_{1-6}$-alkylthio and $R^3$ represents $C_{1-6}$-alkoxy, $C_{1-6}$-alkylthio or pyrrolo or

$R^1$ represents hydrogen; $R^2$ and $R^4$ represent $C_{1-6}$-alkoxy and $R^3$ represents $C_{1-6}$-alkylthio or carboxy-$C_{1-6}$-alkylthio,

and their physiologically compatible salts.

4. 2,4-Diamino-5-benzylpyrimidines of the formula

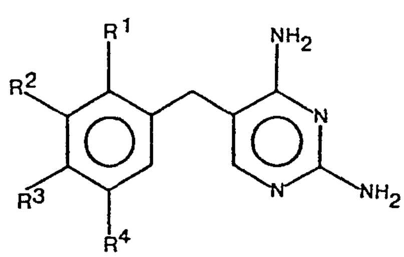

I

wherein

$R^1$ represents hydrogen or $C_{1-6}$-alkyl; $R^2$ represents hydrogen and $R^3$ and $R^4$ represent $C_{1-6}$-alkylthio or

$R^1$ represents hydrogen; $R^2$ and $R^4$ represent $C_{1-6}$-alkylthio and $R^3$ represents $C_{1-6}$-alkoxy, $C_{1-6}$-alkylthio or pyrrolo or

$R^1$ represents hydrogen; $R^2$ and $R^4$ represent $C_{1-6}$-alkoxy and $R^3$ represents carboxy-$C_{1-6}$-alkylthio, and their physiologically compatible salts.

5. 2,4-Diamino-5-benzylpyrimidines of the formula

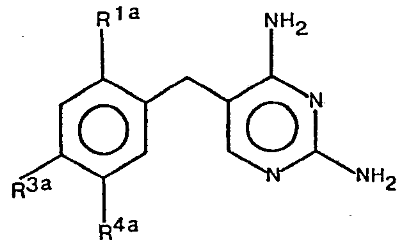

I-1

wherein

$R^{1a}$ signifies hydrogen or $C_{1-6}$-alkyl; $R^{3a}$ signifies $C_{1-6}$-alkylthio and $R^{4a}$ signifies $C_{1-6}$-alkylthio, and their physiologically compatible salts.

6. 2,4-Diamino-5-benzylpyrimidines of the formula

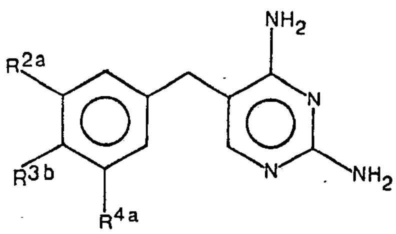

I-2

wherein

$R^{2a}$ signifies $C_{1-6}$-alkylthio; $R^{3b}$ signifies $C_{1-6}$-alkoxy, $C_{1-6}$-alkylthio or pyrrolo and $R^{4a}$ signifies $C_{1-6}$-alkylthio, and their physiologically compatible salts.

7. 2,4-Diamino-5-benzylpyrimidines of the formula

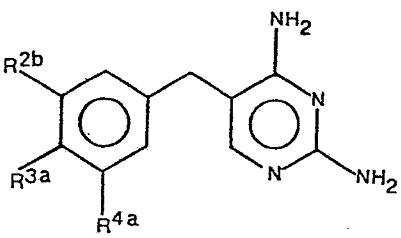

I-3

wherein

$R^{2b}$ and $R^{4b}$ signify $C_{1-6}$-alkoxy and $R^{3c}$ signifies $C_{1-6}$-alkylthio, carboxy-$C_{1-6}$-alkylthio or cyano-$C_{1-6}$-alkylthio, and their physiologically compatible salts.

8. 2,4-Diamino-5-benzylpyrimidines of the formula

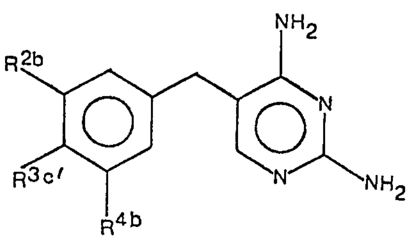

I-3'

wherein

$R^{2b}$ and $R^{4b}$ signify $C_{1-6}$-alkoxy and $R^{3c'}$ signifies carboxy-$C_{1-6}$-alkylthio or cyano-$C_{1-6}$-alkylthio, and their physiologically compatible salts.

9. 2,4-Diamino-5-benzylpyrimidines of the formula

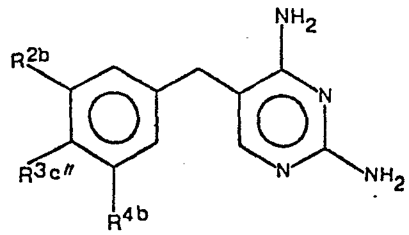

I-3″

wherein

$R^{2b}$ and $R^{4b}$ signify $C_{1-6}$-alkoxy and $R^{3c''}$ signifies $C_{1-6}$-alkylthio or carboxy-$C_{1-6}$-alkylthio, and their physiologically compatible salts.

10. 2,4-Diamino-5-benzylpyrimidines of the formula

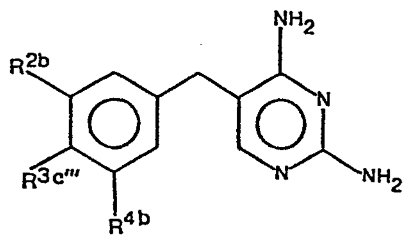

I-3‴

wherein

$R^{2b}$ and $R^{4b}$ signify $C_{1-6}$-alkoxy and $R^{3c'''}$ signifies carboxy-$C_{1-6}$-alkylthio, and their physiologically compatible salts.

11. 2,4-Diamino-5-[3,5-dimethoxy-4-(methylthio)-benzyl]-pyrimidine.

12. 2,4-Diamino-5-[4-(ethylthio)-3,5-dimethoxybenzyl]-pyrimidine.

13. 2,4-Diamino-5-[3,5-bis-(methylthio)-4-pyrrolobenzyl]-pyrimidine.

14. 2,4-Diamino-5-[3,4-bis-(methylthio)-benzyl]-pyrimidine.

15. 2,4-Diamino-5-[3,4,5-tris-(methylthio)-benzyl]-pyrimidine.

16. 2,4-Diamino-5-[2-methyl-4,5-bis-(methylthio)-benzyl]-pyrimidine.

17. 2,4-Diamino-5-[4-methoxy-3,5-bis-(methylthio)-benzyl]-pyrimidine.

18. 2,4-Diamino-5-[4-(4-cyanobutylthio)-3,5-dimethoxybenzyl]-pyrimidine.

19. 2,4-Diamino-5-[4-(carboxymethylthio)-3,5-dimethoxybenzyl]-pyrimidine.

20. Process for the manufacture of compounds in accordance with claim 1 or 2, characterized by (a) condensing a compound of the formula

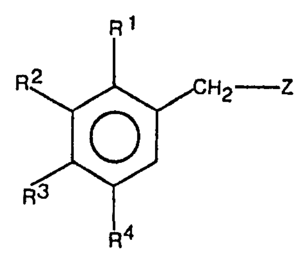

II

wherein

$R^1$, $R^2$, $R^3$ and $R^4$ have the significances given in claim 1 or 2, Z represents a group —C(CN)=CH—Y or —CH(CN)CH($OR^5$)$_2$, Y represents a leaving group and $R^5$ represents $C_{1-6}$-alkyl, 2-$C_{1-6}$-alkoxy-ethyl, 2-phenoxy-ethyl or 2-benzyloxy-ethyl or both residues $R^5$ together represent $C_{1-6}$-alkylene,

with guanidine or a guanidine salt, or

(b) reductively eliminating the substituent X from a compound of the formula

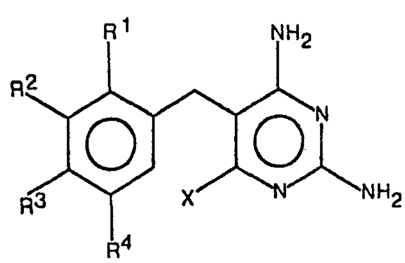

III

wherein

X represents halogen and $R^1$, $R^2$, $R^3$ and $R^4$ have the significances given in claim 1 or 2, or
(c) reacting a substituted benzylpyrimidine of the formula

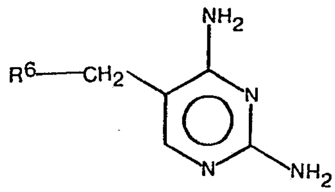

IV

wherein

$R^6$ represents $C_{1-6}$-alkoxy (preferably methoxy), benzyloxy, hydroxy or halogen,

with a compound of the formula

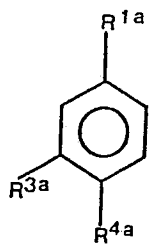

V

wherein

$R^{1a}$, $R^{3a}$ and $R^{4a}$ have the significances given in claim 5,

and, if desired, converting the compound obtained into a physiologically compatible salt.

21. Process for the manufacture of compounds in accordance with claim 3 or 4, characterized by
(a) condensing a compound of the formula

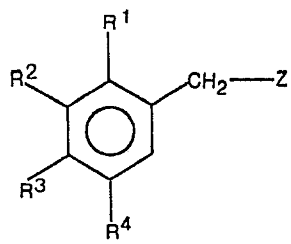

II

wherein

$R^1$, $R^2$, $R^3$ and $R^4$ have the significances given in claim 3 or 4, Z represents a group —C(CN)=CH—Y or —CH(CN)CH($OR^5$)$_2$; Y represents a leaving group and $R^5$ represents $C_{1-6}$-alkyl or both residues $R^5$ together represent $C_{1-6}$-alkylene,

with guanidine or a guanidine salt, or

(b) reductively eliminating the substituent X from a compound of the formula

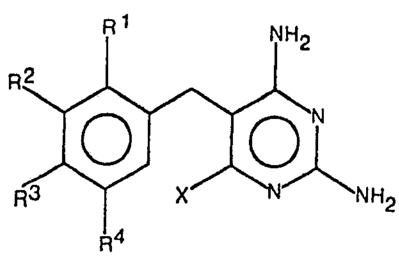

III

wherein

X represents halogen and $R^1$, $R^2$, $R^3$ and $R^4$ have the significances given in claim 3 or 4, or
(c) reacting a substituted benzylpyrimidine of the formula

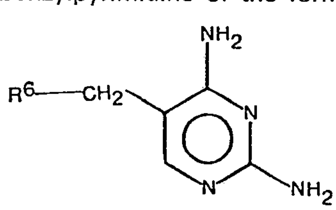

IV

wherein

$R^6$ represents $C_{1-6}$-alkoxy (preferably methoxy), benzyloxy, hydroxy or halogen, with a compound of the formula

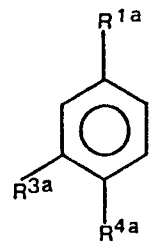

V

wherein

$R^{1a}$, $R^{3a}$ and $R^{4a}$ have the significances given in claim 5,

and, if desired, converting the compound obtained into a physiologically compatible salt.

22. Pharmaceutical preparations based on a compound in accordance with claim 1 or 2.

23. Pharmaceutical preparations based on a compound in accordance with claim 3 or 4.

24. Pharmaceutical preparations based on a compound in accordance with claim 1 or 2 as well as an antibacterially active sulphonamide.

25. Pharmaceutical preparations based on a compound in accordance with claim 3 or 4 as well as an antibacterially active sulphonamide.

26. Process for the manufacture of pharmaceutical preparations in accordance with claims 22—25, characterized by mixing the active substances with non-toxic, inert, solid or liquid carriers which are customary in such preparations and which are suitable for therapeutic administration and bringing the mixture obtained into a suitable galenical form.